# EUROPEAN PATENT APPLICATION

(11) **EP 4 393 935 A1**
(43) Date of publication of application: **03.07.2024**
(21) Application number: 22861353.5
(22) Date of filing: 23.08.2022
(51) Int. Cl.: C07H 19/167, A61K 31/708, A61P 43/00

(54) **METHOD FOR PRODUCING 2?-MODIFIED GUANOSINE COMPOUND**

(30) Priority: 24.08.2021 JP 2021136543
(71) Applicant: Nissan Chemical Corporation, Tokyo 103-6119 (JP)
(72) Inventor: UMEDA, Yasuhiro, Funabashi-shi, Chiba 274-0069 (JP); MIMORI,Yuji, Funabashi-shi, Chiba 274-0069 (JP); MORODOME, Keisuke, Funabashi-shi, Chiba 274-0069 (JP); MATSUOKA, Seiya, Funabashi-shi, Chiba 274-0069 (JP)
(74) Representative: Hoffmann Eitle
(86) International application number: PCT/JP2022/031705
(87) International publication number: WO 2023/027065

(57) **Abstract**

To provide a novel method for producing a 2'-modified guanosine compound of the following formula. 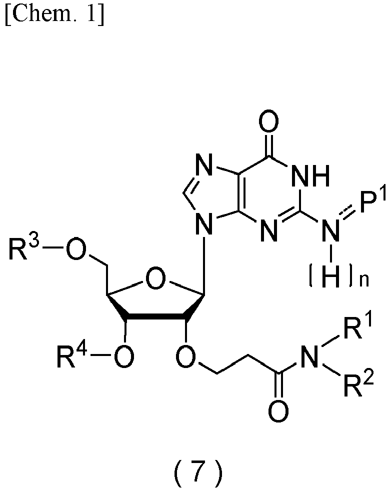 In the formula, R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, n is 0 or 1, when n is 1, P¹ is a protecting group bonded to a nitrogen atom through a single bond, and when n is 0, P¹ is a protecting group bonded to a nitrogen atom through a double bond.

## Description

### TECHNICAL FIELD

The present invention relates to a novel method for producing a 2'-modified guanosine compound.

### BACKGROUND ART

A nucleic acid medicine is a pharmaceutical composed of a nucleic acid (oligonucleotide) forming a complementary base pair with DNA or RNA that is a target and is being expected as a new pharmaceutical. In addition, as nucleic acid units that are used in nucleic acid medicines, a variety of artificial nucleic acid units for which the structure of a natural nucleic acid is changed (artificial nucleosides or artificial nucleotides that are phosphate adducts of artificial nucleosides) have been developed. For example, methylated nucleosides, methoxyethylated nucleosides (MOE) or N-methylcarbamoyl ethylated nucleosides (MCE) of an oxygen atom at a sugar part 2'-position of a ribonucleoside have been reported (for example, refer to Patent Documents 1 and 2 and Non Patent Document 1). It has been reported that, among them, MCE oligonucleotides further improve the nuclease resistance than 2'-position methylated oligonucleotides (for example, refer to Non Patent Document 1).

It has been reported that 2'-(N-substituted or unsubstituted carbamoyl) ethylated nucleosides, such as the MCE, can be produced by a Michael reaction of an acrylic acid ester and a ribonucleoside and subsequent amidation or the like (for example, refer to Patent Document 2 and Non Patent Document 1).

### Related Art Documents

### Patent Documents

[Patent Document 1] JP7-2889A
[Patent Document 2] JP5194256B

### Non Patent Document

[Non Patent Document 1] The Journal of Organic Chemistry, Vol. 76, p. 3042 (2011)

### SUMMARY OF THE INVENTION

### Problem to be Solved by the Invention

Regarding a method for producing a 2'-(N-substituted or unsubstituted carbamoyl) ethylated guanosine compound, a method in which a phenoxyacetyl group is used as a protecting group for a guanine base has been reported (Non Patent Document 1). However, the production method includes complicated purification by column chromatography in many steps and thus has a problem as an industrial production method. An objective of the present invention is to provide a novel method for producing a 2'-(N-substituted or unsubstituted carbamoyl) ethylated guanosine compound that is useful as an industrial production method.

### Means for Solving the Problem

As a result of intensive studies, the present inventors found for the first time a method for producing a 2'-(N-substituted or unsubstituted carbamoyl) ethylated guanosine compound in which a guanine base is protected, the method comprising that the guanosine compound is purified by solid-liquid separation and isolated. Furthermore, the present inventors conducted additional studies based on the above-described knowledge and completed the present invention.

Specifically, the present inventors found a method for producing a 2'-(N-substituted or unsubstituted carbamoyl) ethylated guanosine compound using a 2-aminoadenosine compound in which the 3'-position and the 5'-position are protected as a starting material, the method including i) a Michael reaction with an acrylic acid ester, subsequently, ii) a protection reaction of an amino group of 2-aminoadenosine, iii) an oxidation reaction of a 2-aminoadenine base into a guanine base, iv) a conversion reaction of an ester group introduced into the 2'-position into a N-substituted or unsubstituted carbamoyl group and v) a protection reaction of the guanine base. The production method of the present invention may further include a protection reaction or deprotection reaction of the 3'-position, the 5'-position and/or a nucleic acid base. The present inventors found a useful industrial production method capable of reducing or avoiding purification by column chromatography in the i) to v) as a whole by including a step of purifying a solvate of a 2'-(N-substituted or unsubstituted carbamoyl) ethylated guanosine compound in which a guanine base is protected by solid-liquid separation and isolating the compound. That is, the present invention relates to the followings.

[1] A method for producing a protected guanosine compound of the following formula (6): 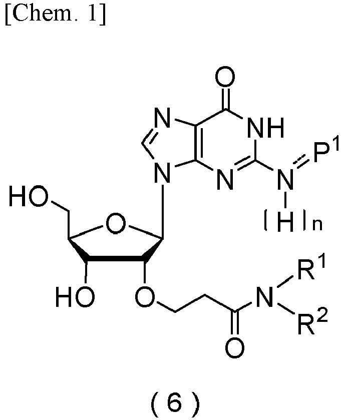
   wherein n is 0 or 1,
   when n is 1, P¹ is a protecting group bonded to a nitrogen atom through a single bond,
   when n is 0, P¹ is a protecting group bonded to a nitrogen atom through a double bond,
   R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups,
   the method comprising steps (e1) and (e2):
      (e1) a step of converting a guanosine compound of the following formula (5) into a protected guanosine compound of formula (6) in a solvent, 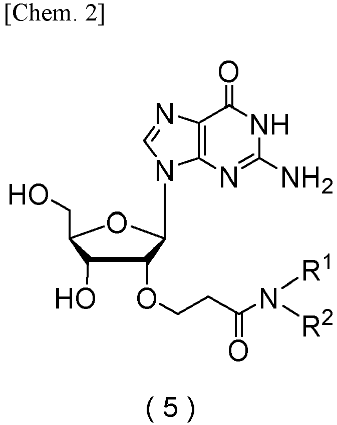 wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups,
      (e2) a step of isolating a solvate of the protected guanosine compound as a solid by solid-liquid separation.
[2] The production method according to [1], further comprising a step of isolating the solvate of the protected guanosine compound of the formula (6) as a crystal by solid-liquid separation.
[3] The production method according to [2], wherein the solvate is a solvate of an organic solvent.
[4] The production method according to [3], wherein the organic solvent is methanol, ethanol, n-propanol or i-propanol.
[5] The production method according to [4], wherein the organic solvent is methanol.
[6] The production method according to [1], wherein n is 1, and P¹ is formula (14), 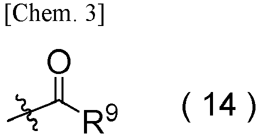 wherein R⁹ is a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group.
[7] The production method according to [1], wherein n is 0, and P¹ is formula (15), 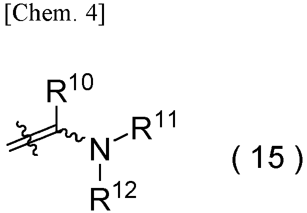
   wherein R¹⁰ is a hydrogen atom, a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group, and
   R¹¹ and R¹² are the same as or different from each other and are substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, or are bonded to each other to form a ring together with the nitrogen atom to which they are bonded.
[8] The production method according to [7], wherein R¹⁰ is a hydrogen atom, and R¹¹ and R¹² are the same as or different from each other and are C₁₋₆ alkyl groups.
[9] The production method according to [7], wherein R¹⁰ is a hydrogen atom, and R¹¹ and R¹² are methyl groups.
[10] The production method according to [1], wherein, when n is 1, P¹ is an acetyl group, a phenoxyacetyl group, a benzoyl group or an i-butyryl group, and, when n is 0, P¹ is a N,N-dimethylaminomethylene group.
[11] The production method according to [1], wherein at least one of R¹ and R² is a hydrogen atom.
[12] The production method according to [1], wherein R¹ is a hydrogen atom, and R² is a methyl group.
[13] The production method according to [1], wherein R¹ and R² are methyl groups.
[14] The production method according to [12], wherein R¹ is a hydrogen atom, R² is a methyl group, n is 0, and P¹ is a N,N-dimethylaminomethylene group.
[15] The production method according to any one of [1] to [14], further comprising the following steps (c) and (d):
   (c) a step of oxidizing a 2-aminoadenosine compound of formula (3) to convert into a guanosine compound of formula (4), 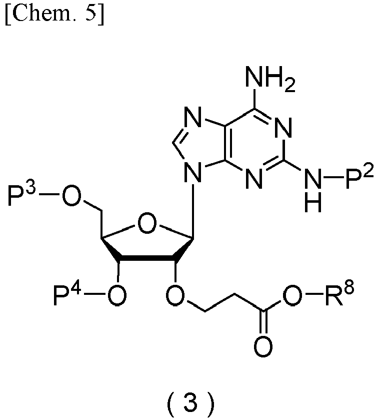
   wherein P² is a protecting group for an amino group, P³ and P⁴ are the same as or different from each other and are protecting groups for a hydroxy group, and R⁸ is a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group, 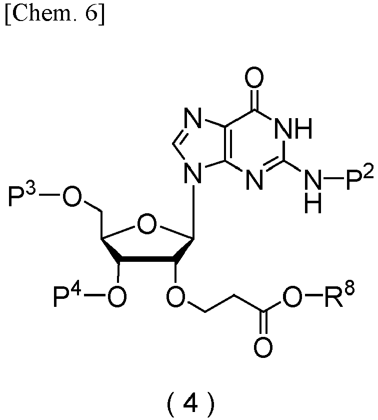
   wherein P², P³, P⁴ and R⁸ are the same definition as the one defined in formula (3), (d) a step of converting the guanosine compound of formula (4) into a compound of formula (5), 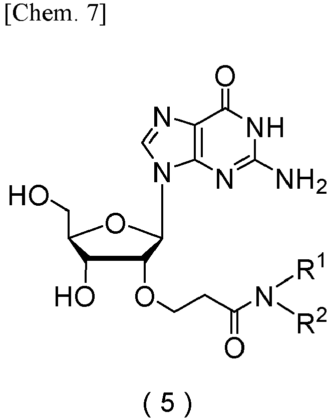
   wherein R¹ and R² are the same definition as the one defined in formula (5).
[16] The production method according to [15], wherein the step (d) is any of a step (d1), (d2) or (d3):
   (d1) a step including:
      a step of reacting the compound of formula (4) and an amino compound of the following formula (20) to obtain an amide compound, 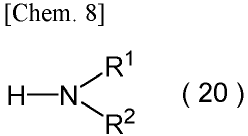
      wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl group or substitutable C₆₋₁₄ aryl groups, and a step of deprotecting at least one of P², P³ and P⁴ in the amide compound,
   (d2) a step including:
      a step of hydrolyzing the guanosine compound of formula (4) to obtain a carboxylic acid compound, a step of condensing the hydrolyzed carboxylic acid compound and the amino compound of formula (20) to obtain an amide compound, 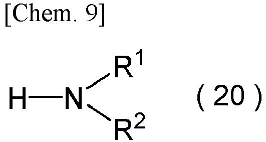
      wherein R¹ and R² are the same definition as the one defined in formula (20), and
      a step of deprotecting at least one of P², P³ and P⁴ in the amide compound,
   (d3) a step including:
      a step of deprotecting at least one of P², P³ and P⁴ of the compound of formula (4),
      a step of reacting the deprotected compound and the amino compound of formula (20) to obtain an amide compound, 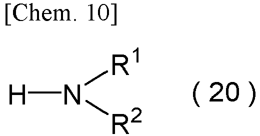
      wherein R¹ and R² are the same definition as the one defined in formula (20), and a step of deprotecting at least one of P², P³ and P⁴ in the amide compound.
[17] The production method according to [15], wherein P³ and P⁴ together form the following formula (19), 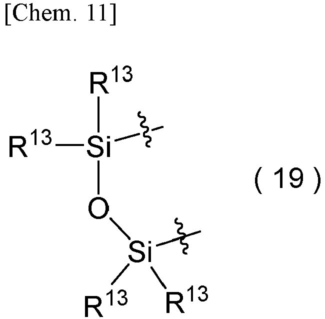 wherein R¹³s are the same as or different from one another and are substitutable C₁₋₆ alkyl groups or substitutable phenyl groups.
[18] The production method according to [17], wherein R¹³ is an i-propyl group.
[19] The production method according to [15], wherein R⁸ is a methyl group, an ethyl group, a n-propyl group or an i-propyl group.
[20] The production method according to [15], wherein P² is an acetyl group, a propionyl group, an i-butyryl group, a pivaloyl group, a n-butyryl group, a benzoyl group or a phenoxyacetyl group.
[21] The production method according to [15], wherein P² is an i-butyryl group.
[22] The production method according to [15], further comprising the following step (b):
   (b) a step of protecting a 2-amino group of a 2-aminoadenosine compound of the following formula (2) to convert into a compound of formula (3), 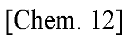 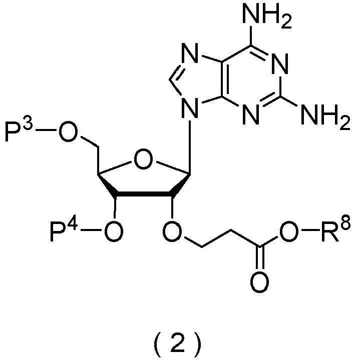
   wherein P³ and P⁴ are the same as or different from each other and are protecting groups for a hydroxy group, and R⁸ is a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group.
[23] The production method according to [22], further comprising the following step (a):
   (a) a step of reacting an acrylic acid ester of the following formula (9) with the 2-aminoadenosine compound of formula (1) to convert into the 2-aminoadenosine compound of formula (2), 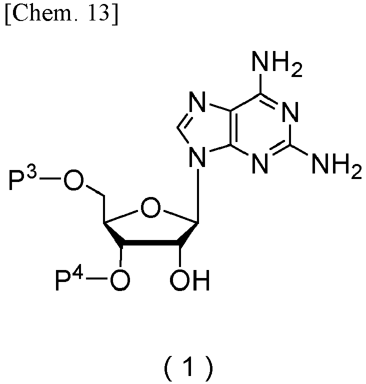
   wherein P³ and P⁴ are the same as or different from each other and are protecting groups for a hydroxy group, 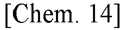 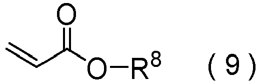
   wherein R⁸ is a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group, 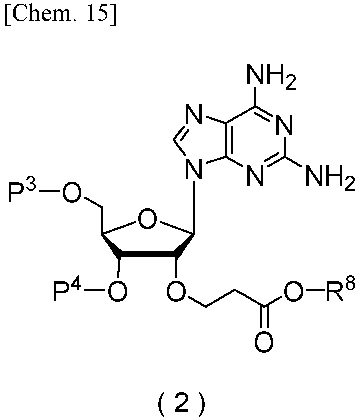
   wherein P³ and P⁴ are the same as or different from each other and are protecting groups for a hydroxy group, and R⁸ is a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group.
[24] The production method according to [23], wherein the step (a), the step (b), the step (c), the step (d), the step (e1) and the step (e2) as a whole include a column purification step once to three times, or do not include a column purification step.
[25] The production method according to [24], wherein the step (a), the step (b), the step (c), the step (d), the step (e1) and the step (e2) are continuously performed.
[26] A method for producing a guanosine compound of formula (7): 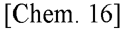 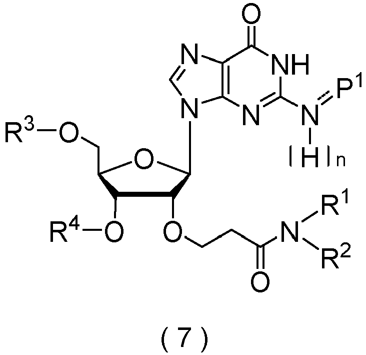
   wherein n is 0 or 1,
   when n is 1, P¹ is bonded to a nitrogen atom through a single bond,
   when n is 0, P¹ is a protecting group bonded to a nitrogen atom through a double bond,
   R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups,
   R³ is a protecting group for a hydroxy group, and
   R⁴ is a hydrogen atom, a protecting group for a hydroxy group or a group of formula (17): 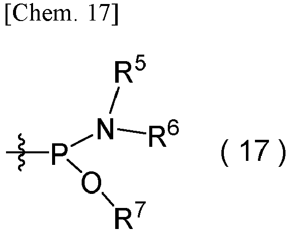
   wherein R⁵ and R⁶ are the same as or different from each other and are C₁₋₆ alkyl groups, or R⁵ and R⁶ are bonded to each other to form a ring together with the nitrogen atom to which they are bonded, and R⁷ is a protecting group for a phosphate group, the method comprising:
      a step of converting a guanosine compound of the following formula (5) into a protected guanosine compound of the following formula (6), 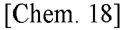 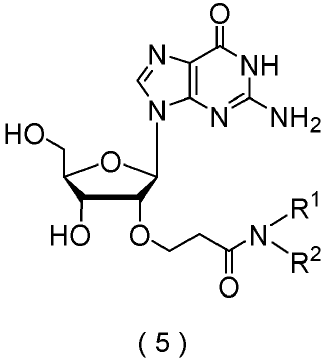
      wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, 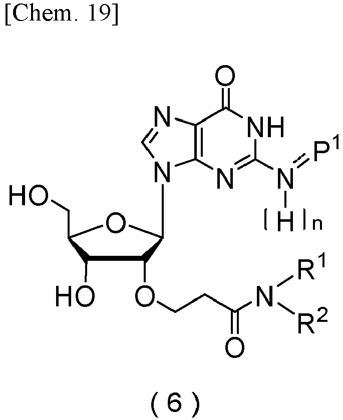
      wherein n is 0 or 1,
      when n is 1, P¹ is bonded to a nitrogen atom through a single bond,
      when n is 0, P¹ is a protecting group bonded to a nitrogen atom through a double bond,
      R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups;
      a step of isolating a solvate of the protected guanosine compound as a solid; and
      a step of dissolving the solvate of the protected guanosine compound in a solvent and protecting a 5'-position hydroxy group.
[27] The production method according to [26], wherein R³ is a 4,4'-dimethoxytriphenylmethyl group.
[28] The production method according to [26], wherein R⁷ is a 2-cyanoethyl group.
[29] The production method according to [28], wherein R⁵ and R⁶ are i-propyl groups.
[30] A method for producing a compound of formula (11), comprising:
   a step of isobutylating a 2-amino group of a compound of the following formula (21) to convert into a compound of the following formula (11); and 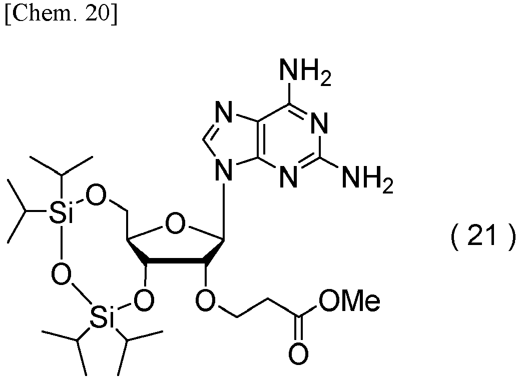 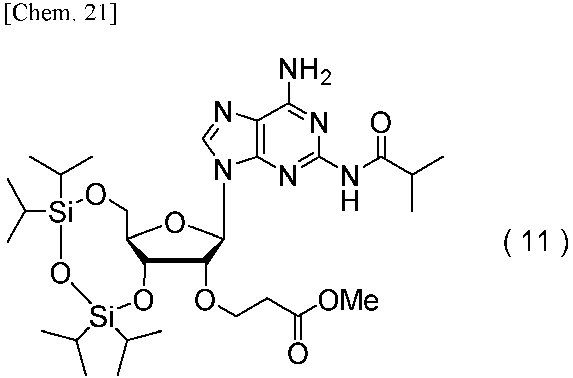
   a step of isolating the compound of formula (11) as a solid by solid-liquid separation.
[31] A method for producing a compound of formula (5), comprising:
   a step of deprotecting an isobutyryl group and a silyl group of a compound of the following formula (11): 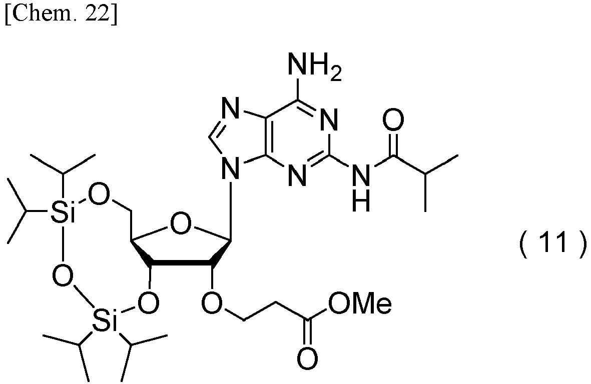
   a step of adding an amino compound of the following formula (20) in a solvent to convert into a compound of the following formula (5), 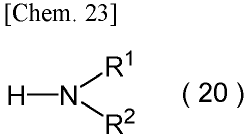
   wherein R¹ and R² are the same definition as the one in formula (20) of [16], 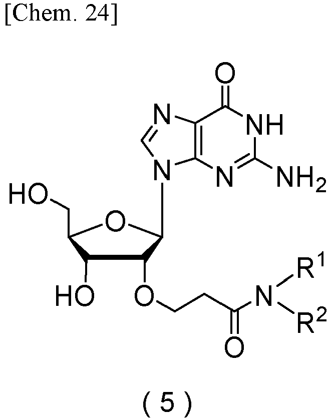
   wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups; and
   a step of isolating the compound of formula (5) as a solid by solid-liquid separation.
[32] A solvate of a protected guanosine compound of the following formula (6): 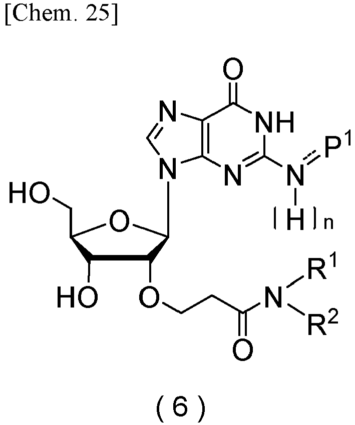
   wherein n is 0 or 1,
   when n is 1, P¹ is a protecting group bonded to a nitrogen atom through a single bond, when n is 0, P¹ is a protecting group bonded to a nitrogen atom through a double bond,
   R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups.
[33] The solvate according to [32], wherein the solvate is a methanol solvate.

### Effects of the Invention

The present invention made it possible to provide a novel method for producing a 2'-(N-substituted or unsubstituted carbamoyl) ethylated guanosine compound. According to the present invention, in a method for producing a 2'-(N-substituted or unsubstituted carbamoyl) ethylated guanosine compound using a 2-aminoadenosine compound in which the 3'-position and the 5'-position are protected as a starting material, complicated purification by column chromatography can be reduced or avoided. The present invention made the industrial production of a 2'-(N-substituted or unsubstituted carbamoyl) ethylated guanosine compound possible and made the mass production of the compound possible.

In addition, it is possible to obtain a high-purity 2'-(N-substituted or unsubstituted carbamoyl) ethylated guanosine compound or a solvate thereof.

### BRIEF DESCRIPTION OF THE DRAWINGS

[FIG. 1] FIG. 1 is a powder X-ray diffraction diagram of a compound described in Example 1.
[FIG. 2] FIG. 2 is a powder X-ray diffraction diagram of a compound described in Example 2.
[FIG. 3] FIG. 3 is a powder X-ray diffraction diagram of a compound described in Example 3.

### MODES FOR CARRYING OUT THE INVENTION

Hereinafter, the present invention will be described in more detail. In the present invention, "n-" means normal, "i-" means iso, "s-" and "sec-" mean secondary, "t-" and "tert-" mean tertiary, "o-" means ortho, "m-" means metha, "p-" means para, "Ph" means phenyl, "Bu" means butyl, "Ts" means p-toluenesulfonyl, and "Bn" means benzyl.

First, terms that are used to describe chemical structures in the present specification will be described.

"C₁₋₆ alkyl group" means a linear or branched alkyl group having 1 to 6 carbon atoms, and specific examples thereof include methyl group, ethyl group, n-propyl group, i-propyl group, n-butyl group, i-butyl group, s-butyl group, t-butyl group, n-pentyl group, 2-methylbutyl group, 3-methylbutyl group, n-hexyl group, 2-methylpentyl group, 3-methylpentyl group, 4-methylpentyl group, 2-ethylbutyl group and 3-ethylbutyl group.

"C₇₋₁₆ aralkyl group" means an alkyl group having an aromatic hydrocarbon as a substituent and having 7 to 16 carbon atoms in the entire substituent. Specific examples thereof include phenylmethyl group (benzyl group), 1-phenylethyl group, 2-phenylethyl group, 1-phenylpropyl group, 2-phenylpropyl group, 3-phenylpropyl group, naphthalen-1-ylmethyl group, naphthalen-2-ylmethyl group, naphthalen-1-ylethyl group, naphthalen-2-ylethyl group, anthracen-1-ylmethyl group, anthracen-2-ylmethyl group and anthracen-9-ylmethyl group.

"C₂₋₆ alkenyl group" means a linear or branched alkenyl group having 2 to 6 carbon atoms and having at least one double bond, and specific examples thereof include ethenyl group, 1-propenyl group, 2-propenyl group, 1-butenyl group, 2-butenyl group, 3-butenyl group, 1-pentenyl group, 2-pentenyl group, 3-pentenyl group and 4-pentenyl group.

"C₂₋₆ alkynyl group" means a linear or branched alkynyl group having 2 to 6 carbon atoms and having at least one triple bond, and specific examples thereof include ethynyl group, 1-propynyl group, 2-propynyl group, 1-butynyl group, 2-butynyl group, 3-butynyl group, 1-pentynyl group, 2-pentynyl group, 3-pentynyl group and 4-pentynyl group.

"C₆₋₁₄ aryl group" means an aromatic hydrocarbon group having 6 to 14 carbon atoms, and specific examples thereof include phenyl group, 1-naphthyl group, 2-naphthyl group, 1-anthracenyl group, 2-anthracenyl group and 9-anthracenyl group.

"Substitutable" means unsubstituted or substituted with any number of any substituents. "Substituted" means substituted with any number of any substituents. Any number is, for example, one to three. The above-mentioned "any substituent" is not particularly limited as long as a substituent does not adversely affect the present reaction. Examples of any substituent include a halogen atom, hydroxy group, a protective form of hydroxy group, amino group, a protective form of amino group, carboxy group, a protective form of carboxy group, a nitro group, a cyano group, C₁₋₆ alkyl group, C₂₋₆ alkenyl group, C₆₋₁₄ aryl group, and C₇₋₁₆ aralkyl group and the like and further include phenyl group substituted with the above-listed substituent.

"Bonded to each other to form a ring together with the nitrogen atom to which they are bonded" means that two substituents bonded to one nitrogen atom are bonded to each other to form a five to seven-membered ring together with the nitrogen atom. Specific examples of the five to seven-membered ring include pyrrolidine, piperidine, azepine, morpholine and homomorpholine.

A halogen atom means a fluorine atom, a chlorine atom, a bromine atom or an iodine atom.

"Protecting group for hydroxy group" refers to a protecting group that is normally used in organic synthetic chemistry (particularly, nucleic acid synthesis). Examples thereof include C₁₋₆ alkyl group (methyl group, ethyl group, tert-butyl group or the like), allyl group, benzyl-based protecting group (benzyl group, p-methoxybenzyl group or the like), acyl-based protecting group (formyl group, acetyl group, benzoyl group or the like), acetal-based protecting group (methoxymethyl group, ethoxymethyl group, 2-tetrahydropyranyl group or the like), silyl-based protecting group (trimethylsilyl group, triisopropylsilyl group, t-butyldimethylsilyl group, t-butyldiphenylsilyl group or the like), triarylmethyl-based protecting group (triphenylmethyl group, 4,4'-dimethoxytriphenylmethyl group or the like), but are not limited thereto. A protecting group for a hydroxy group that is particularly preferable in each step will be described below.

"Protecting group for amino group" refers to a protecting group that is normally used in organic synthetic chemistry (particularly, nucleic acid synthesis). Examples thereof include acyl-based protecting group (formyl group, acetyl group, benzoyl group, isobutyryl group, pivaloyl group, trifluoroacetyl group, phenoxyacetyl group or the like), carbamate-based protecting group (tert-butoxycarbonyl group, benzyloxycarbonyl group, 9-fluorenylmethyloxycarbonyl group or the like), sulfone-based protecting group (mesyl group, p-toluenesulfonyl group, 2-nitrobenzenesulfonyl group or the like), triarylmethyl-based protecting group (triphenylmethyl group, 4,4'-dimethoxytriphenylmethyl group or the like), amidinyl-based protecting group (N,N-dimethylaminomethylene group, N,N-diethylaminomethylene group, N,N-diisopropylaminomethylene group, 1-pyrrolidinomethylene group or the like), but are not limited thereto. A protecting group for a hydroxy group that is particularly preferable in each step will be described below.

"Protecting group for phosphate group" refers to a protecting group that is normally used in organic synthetic chemistry (particularly, nucleic acid synthesis). Examples thereof include alkyl-based protecting group (2-cyanoethyl group, 2,2,2-trichloroethyl group or benzyl group), aryl-based protecting group (2-chlorophenyl group or 4-chlorophenyl group) and the like, but are not limited thereto. The protecting group for a phosphate group can also be similarly used in phosphite diesters or phosphite monoamide forms.

"Deprotection" means the removal of a protecting group that is used in normal organic synthetic reactions. From a protected hydroxy group, a protected amino group, a protected phosphate group and a protected carboxy group, a protecting group can be removed by performing a deprotection reaction (for example, refer to Protective Groups in Organic Synthesis, Fourth edition, written by T. W. Greene, John Wiley & Sons Inc. (2006) or the like).

The kind of a base that is used in the present reaction is not limited as long as the base does not affect the reaction, and examples thereof include triethylamine, N,N-diisopropylamine, N-methylmorpholine, pyridine, 1,8-diazabicyclo[5.4.0]-7-undecene, sodium carbonate, potassium carbonate, cesium carbonate, sodium hydroxide, potassium hydroxide, cesium hydroxide, sodium methoxide, potassium methoxide, sodium tert-butoxide, potassium tert-butoxide and the like. A base that is particularly preferable in each step will be described below.

One base may be used singly or two or more bases may be used in combination.

A solvent that is used in the present invention is not limited as long as the solvent does not impair the progress of a reaction, and preferable examples thereof include solvents such as an aprotic polar organic solvent (for example, N,N-dimethylformamide, dimethylsulfoxide, N,N-dimethylacetamide, tetramethylurea, sulfolane, N-methylpyrrolidone, N,N-dimethylimidazolidinone or the like), an ether-based solvent (for example, diethyl ether, diisopropyl ether, t-butyl methyl ether, tetrahydrofuran, dioxane or the like), an aliphatic hydrocarbon-based solvent (for example, pentane, n-hexane, c-hexane, octane, decane, decalin, petroleum ether or the like), an aromatic hydrocarbon-based solvent (benzene, chlorobenzene, o-dichlorobenzene, nitrobenzene, toluene, xylene, mesitylene, tetralin or the like), a halogenated hydrocarbon-based solvent (for example, chloroform, dichloromethane, dichloroethane, carbon tetrachloride or the like), a lower aliphatic acid ester-based solvent (for example, methyl acetate, ethyl acetate, butyl acetate, methyl propionate or the like), an alkoxyalkane-based solvent (for example, dimethoxyethane, diethoxyethane or the like), an alcoholic solvent (for example, methanol, ethanol, n-propanol, i -propanol, n-butanol, tert-butanol or the like), acetic acid and water. A solvent that is particularly preferable in each step will be described below.

One solvent may be used singly or a solvent mixture of two or more solvents may be used.

"Solid-liquid separation" refers to an operation of separating a solid that disperses in a liquid from the liquid. Specific examples of the operation include filtration, centrifugation and the like, and filtration is preferable.

"Crystal" refers to a solid in which atoms or molecules are regularly arranged. For example, the crystal can be confirmed by detecting a characteristic peak with a powder X-ray diffractometer. In the powder X-ray diffractometer, a characteristic peak can be detected with an error of approximately ±0.2°.

"Solvate" refers to a molecular cluster in which a compound is tied to a solvent molecule of water, an organic solvent or the like. A target compound and a solvent molecule are tied to each other by an electrostatic force or a hydrogen bond and can be present in a variety of ratios.

Hereinafter, the present invention will be described in detail. In drawings shown below until examples, R¹ to R¹³ and P¹ to P⁴ in formulae are the same meanings as described above.

A method for producing a series of compounds of the present invention will be shown in the following schemes. In the schemes, "step" means steps. In detailed description to be described below, "compound of formula (formula number)" will be referred to as "compound (formula number) in some cases. For example, a compound of formula (1) is referred to as a compound (1).

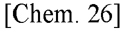

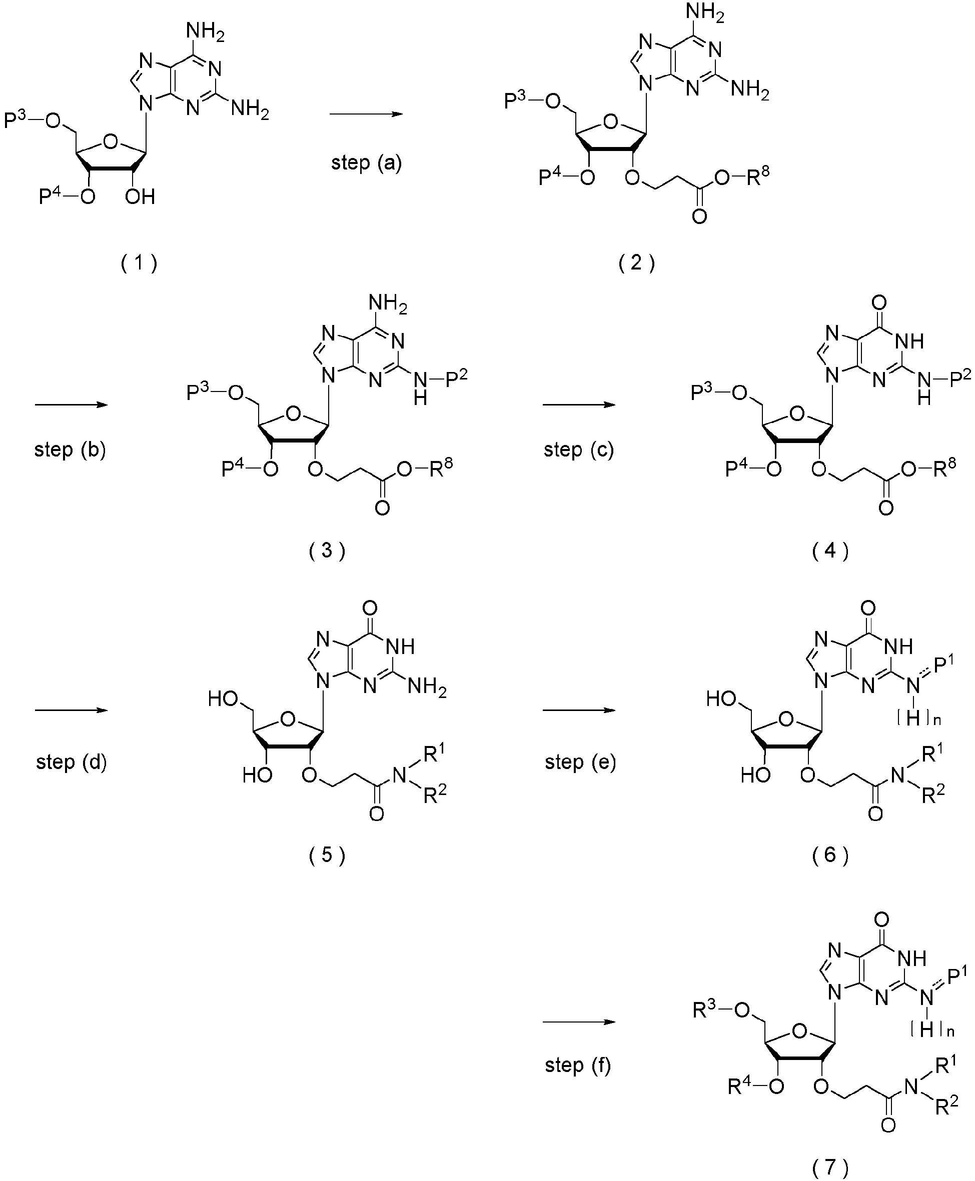

A compound (1) that is used in the present invention can be obtained by, as shown in the following scheme, 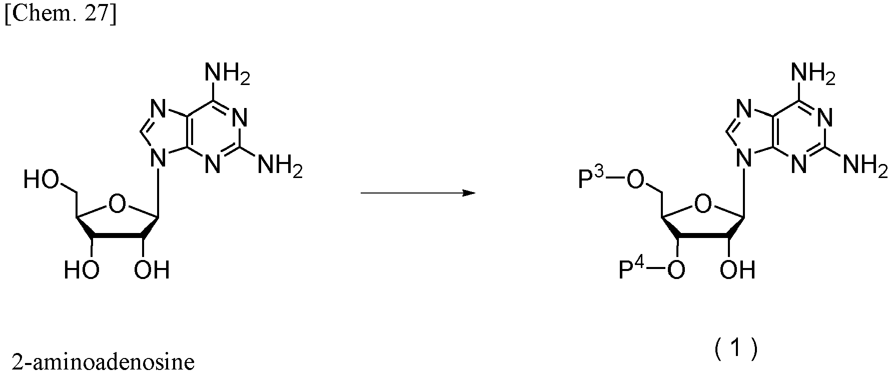 protecting a 3' hydroxy group and a 5' hydroxy group of a 2-aminoadenosine. As protecting groups for the hydroxy groups, as described above, protecting groups that are normally used in organic synthetic chemistry (particularly, nucleic acid synthesis) may be used. P³ and P⁴ may be each independently a group that protects the 5' hydroxy group and the 3' hydroxy group, or P³ and P⁴ may together form a group that protects the 3' hydroxy group and the 5' hydroxy group as shown in the following formula (19). 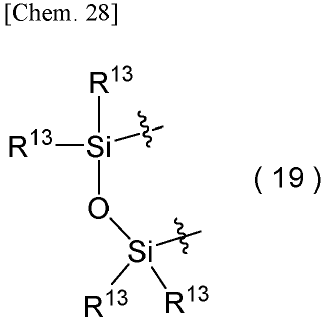 wherein R¹³s are the same as or different from one another and are substitutable C₁₋₆ alkyl groups or substitutable phenyl groups. R¹³s are the same as or different from one another and are preferably C₁₋₆ alkyl groups and more preferably isopropyl groups. The upper wavy line in formula (19) indicates bonding to an oxygen atom to which P³ is bonded, and the lower wavy line indicates bonding to an oxygen atom to which P⁴ is bonded. The compound (1) of formula (19) having a protecting group can be synthesized by a method described in Non Patent Document 1 or a method according thereto.

### Step (a): Michael reaction step

By reacting an acrylic acid ester (9) on the 2' hydroxy group of the compound (1), 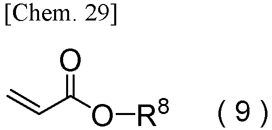 a compound (2) can be synthesized. The acrylic acid ester can be commercially available product or can be synthesized by a method known by a person skilled in the art.

A substituent R⁸ in the acrylic acid ester (9) that can be used in the present reaction is a substitutable C₁₋₆ alkyl group, a substitutable C₇₋₁₆ aralkyl group, a substitutable C ₂₋₆ alkenyl group, a substitutable C₂₋₆ alkynyl group or a substitutable C₆₋₁₄ aryl group. As R⁸, a C₁₋₆ alkyl group is preferable, methyl group, ethyl group, n-propyl group or i-propyl group is more preferably, and methyl group is still more preferable.

The amount of the acrylic acid ester used is also dependent on the kind of the substituent R⁸ and is preferably 1 to 100 molar equivalents, more preferably 5 to 50 molar equivalents and still more preferably 10 to 30 molar equivalents with respect to the molar equivalent of the compound (1).

In the present reaction, a base is preferably used. As the kind of the base that is used in the present reaction, a carbonate metal (potassium carbonate, cesium carbonate or the like) or a metal alkoxide (potassium tert-butoxide or the like) is preferable, and cesium carbonate is more preferable.

The amount of the base is preferably 0.2 to 5 molar equivalents, more preferably 0.5 to 2 molar equivalents and still more preferably 0.8 to 1.2 molar equivalents with respect to the molar equivalent of the compound (1).

As a solvent that is used in the present reaction, an ether-based solvent or an alcoholic solvent is preferable, an alcoholic solvent is more preferable, and tert-butanol is still more preferable.

The reaction temperature is preferably 0 to 40°C and more preferably 20 to 30°C.

The reaction time is, for example, 1 to 48 hours, preferably 4 to 36 hours and more preferably 8 to 24 hours.

### Step (b): Protection step of 2-aminoadenine

A 2-amino group of the compound (2) is protected, and a compound (3) can be synthesized. For the protection of the 2-amino group, as described above, a protecting group that is often used in organic synthetic chemistry (for example, nucleic acid chemistry) can be used, and the 2-amino group can be protected by, for example, a method described in Protective Groups in Organic Synthesis, Fourth edition, written by T. W. Greene, John Wiley & Sons Inc. (2006). As the protecting group for the amino group, phenoxyacetyl group, 4-isopropylphenoxyacetyl group, acetyl group, benzoyl group, isobutyryl group or N,N-dimethylaminomethylene group is preferable.

For example, when the protecting group is an acyl-based protecting group (for example, isobutyryl, acetyl, benzoyl or phenoxyacetyl), the 2-amino group can be protected by reacting a corresponding acyl halide (for example, isobutyryl chloride, acetyl chloride, benzoyl chloride or phenoxyacetyl chloride) in a solvent under a basic condition (for example, triethylamine, N',N'-diisopropylethylamine or pyridine). The solvent is not particularly limited as long as a protection reaction progresses favorably, an ether-based solvent such as tetrahydrofuran, a halogenated hydrocarbon-based solvent such as dichloromethane or a lower aliphatic acid ester-based solvent such as ethyl acetate is preferable, and ethyl acetate is more preferable.

The temperature is preferably -20 to 20°C and more preferably -10 to 10°C.

The reaction time is, for example, 0.5 to 48 hours, preferably 1 to 24 hours and more preferably 1 to 6 hours.

In the protection step of a 2-aminoadenine, there are cases where, as shown in the following scheme, 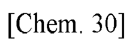 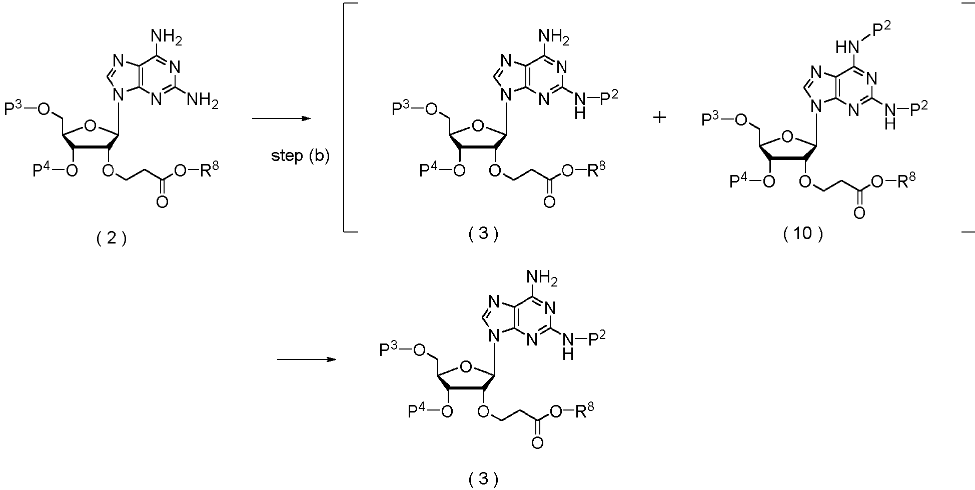 not only a 2-amino group is protected, but a 6-amino group is also protected at the same time, and a compound (10) in which the two amino groups are protected is generated. In such a case, a protecting group for the 6-amino group in the compound (10) is deprotected, and the compound (3) can also be synthesized. For example, in a case where the protecting group for an amino group indicated by P² is an acyl-based protecting group (for example, isobutyryl, acetyl, benzoyl or phenoxyacetyl), the protecting group for the 6-amino group can be deprotected by stirring in a solvent under a basic condition (for example, triethylamine, N,N-diisopropylethylamine, 1,8-diazabicyclo[5.4.0]-7-undecene or pyridine). The solvent is preferably an alcoholic solvent and more preferably methanol. Each reference sign in the compound (10) represents the same meaning as each reference sign in the compound (3).

The reaction temperature in removing the protecting group for the 6-amino group for deprotection varies with the protecting group or reaction conditions, but is preferably -20°C to a solvent reflux temperature and more preferably -10 to 30°C. The reaction time is, for example, 0.5 to 48 hours, preferably 1 to 24 hours and more preferably 1 to 6 hours.

The compound (3) can be obtained as a solid (preferably as a crystal) by filtering a precipitated solid in a solvent and performing solid-liquid separation.

The solvent that is used in the solid-liquid separation is not particularly limited as long as the compound (3) is precipitated as a solid and a small amount of the remaining raw material or a by-product is dissolved in the solvent, and examples thereof include ether-based solvents, aliphatic hydrocarbon-based solvents, lower aliphatic acid ester-based solvents, alcoholic solvents, water and the like. An alcoholic solvent is preferable, methanol, ethanol, n-propanol or i-propanol is more preferable, and methanol is still more preferable.

Two or more of the solvents can be used in combination.

The temperature is not particularly limited as long as the compound (3) is precipitated and an impurity such as the remaining raw material or the by-product is dissolved at the temperature.

For example, in a case where the compound (3) is a compound of formula (11), 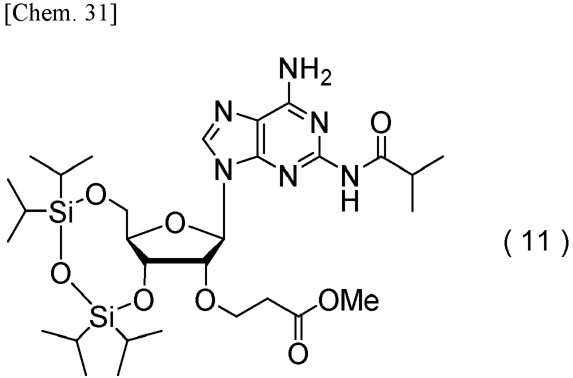 the compound can be obtained as a solid, preferably as a crystal, by stirring the compound in a solvent mixture of methanol and water and separating a precipitated solid into solid and liquid by filtration. The volume ratio (methanol/water) of the solvent mixture of methanol and water is not particularly limited as long as the compound (3) is precipitated and the remaining raw material, the by-product or the like is dissolved and is 5/1 to 1/1, more preferably 3/1 to 1/1 and still more preferably 1.5/1 to 2.5/1.

The stirring temperature is preferably 0 to 40°C and more preferably 20 to 30°C.

### Step (c): Oxidation step

A compound (4) can be synthesized by oxidizing the compound (3). The compound (3) can be oxidized by adding an oxidant or an oxidation enzyme to the compound (3) in a solvent.

As the oxidant, nitrous acid or metal salt of nitrous acid such as nitrous acid, sodium nitrite or potassium nitrite can be used. Sodium nitrite is preferable.

The amount of the oxidant used is preferably 1.0 to 10 molar equivalents, more preferably 2.0 to 7.0 molar equivalents and still more preferably 3.0 to 5.0 molar equivalents with respect to 1 molar equivalent of the compound (3).

In the step (c), an acidic solvent is preferably used. Hydrochloric acid, a sulfuric acid aqueous solution, a nitric acid aqueous solution or an acetic acid aqueous solution is more preferable, and an acetic acid aqueous solution is still more preferable. It is also possible to combine a solvent such as tetrahydrofuran.

The reaction temperature is preferably 20 to 80°C and more preferably 40 to 60°C.

The reaction time is, for example, 0.5 to 48 hours, preferably 1 to 24 hours and more preferably 2 to 6 hours.

In the step (c), adenosine deaminase can be used as the oxidation enzyme. For example, compound (4) can be synthesized by a method described in pp. 4127 to 4132 of Bioorganic & Medicinal Chemistry Letters, 2012, Vol. 22. As the solvent, for example, an acidic phosphate buffer is used.

Adenosine deaminase can be purchased from, for example, Sigma-Aldrich Co. LLC.

### Step (d) amidation step and deprotection step

A compound (5) can be synthesized by performing the conversion of an ester group in the compound (4) into a N-substituted or unsubstituted carbamoyl group and the deprotection of P², P³ and P⁴.

As the order of each reaction, the conversion of the ester group into a N-substituted or unsubstituted carbamoyl group and then the deprotection can be performed in this order or can be performed in reverse order. It is also possible to perform the deprotection for one or two of P², P³ and P⁴ before the conversion of the ester group into a N-substituted or unsubstituted carbamoyl group and perform the deprotection for the remaining two or one after the conversion. Two or three of P², P³ and P⁴ can also be deprotected at the same time. For example, when P³ and P⁴ together form the group of formula (19), P³ and P⁴ can be deprotected at the same time.

In addition, it is also possible to perform part or all of the deprotection of P², P³ and P⁴ at the same time as the conversion of the ester group into a N-substituted or unsubstituted carbamoyl group.

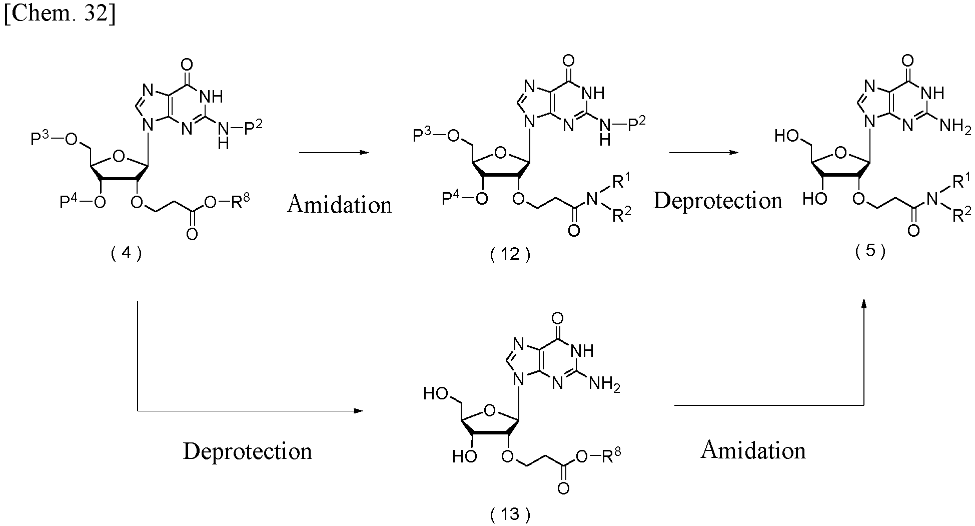

R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups. At least one of R¹ and R² is preferably a hydrogen atom, and it is more preferable that R¹ is a hydrogen atom and R² is a C₁₋₆ alkyl group. It is still more preferable that R¹ is a hydrogen atom and R² is a methyl group. In addition, it is also preferable that R¹ and R² are both methyl groups. Each reference sign in the compounds (12) and (13) represents the same meaning as each reference sign in the compound (4) and/or (5).

The conversion of an ester group in the compound (4) or the compound (13) into a N-substituted or unsubstituted carbamoyl group can be performed by reacting a corresponding amino compound with the ester group. The corresponding amino compound is the following formula (20). 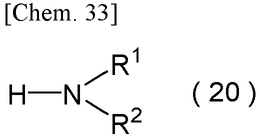 wherein R¹ and R² are the same definition as the one defined in formula (20).

The amount of the amino compound used varies with the kind, but is preferably 1.0 to 100 molar equivalents, more preferably 5.0 to 50 molar equivalents and still more preferably 10 to 30 molar equivalents with respect to 1 molar equivalent of the compound (4) or the compound (13), which serves as a raw material.

As a solvent that is used in the present reaction, an alcoholic solvent is preferable, and methanol is more preferable.

The reaction temperature is preferably 0°C to a solvent reflux temperature and more preferably 20 to 40°C.

The reaction time is, for example, 0.5 to 48 hours, preferably 1 to 36 hours and more preferably 4 to 24 hours.

Regarding the conversion of the ester group in the compound (4) or the compound (13) into a N-substituted or unsubstituted carbamoyl group, the N-substituted or unsubstituted carbamoyl group can also be synthesized by hydrolyzing the ester group and performing a condensation reaction between the obtained carboxylic acid and a corresponding amino compound. The hydrolysis and the condensation reaction can be performed using conditions that are generally used in organic synthetic chemistry.

The hydrolysis can be performed by, for example, reacting a reagent such as a metal hydroxide such as potassium hydroxide or sodium hydroxide in a solvent. The solvent is preferably an alcoholic solvent and more preferably methanol or ethanol. The reaction temperature is preferably -20°C to a solvent reflux temperature and more preferably 0 to 40°C. The reaction time is, for example, 0.5 to 48 hours, preferably 1 to 24 hours and more preferably 1 to 6 hours.

The condensation reaction can be performed by, for example in a solvent, reacting the carboxylic acid obtained by the hydrolysis and the corresponding amino compound in the presence of a condensing agent (for example, N,N'-dicyclohexylcarbodiimide, 1-ethyl-3-(3-dimethylaminopropyl)carbodiimide hydrochloride or the like). The solvent is preferably a halogenated hydrocarbon-based solvent and more preferably dichloromethane. The reaction temperature is preferably -20°C to a solvent reflux temperature and more preferably 0 to 20°C. The reaction time is, for example, 0.5 to 48 hours, preferably 1 to 24 hours and more preferably 1 to 6 hours.

The deprotection of 3',5' hydroxy groups and the 2-amino group in the compound (4) or the compound (12) can be performed under conditions described in, for example, Protective Groups in Organic Synthesis, Fourth edition, written by T. W. Greene, John Wiley & Sons Inc. (2006).

For example, the deprotection of the 3',5' hydroxy groups can be performed by reacting a reaction substrate with a fluorine compound (for example, ammonium fluoride, tetrabutylammonium fluoride, triethylamine trihydrofluoride salt or the like) in a solvent. The solvent is preferably an alcoholic solvent and more preferably methanol. The reaction temperature is preferably -20°C to a solvent reflux temperature, more preferably 0 to 60°C and still more preferably 20 to 40°C. The reaction time is, for example, 0.5 to 48 hours, preferably 1 to 36 hours and more preferably 1 to 24 hours.

As the detail of a preferable order or the combination of the kinds of the reactions of the conversion reaction of the ester group into a N-substituted or unsubstituted carbamoyl group and the removal of P², P³ and P⁴ for deprotection in the step (d), the step (d) is, for example, any of steps (d1), (d2), (d3) and (d4).

The step (d1) is a step including
a step of reacting the compound (4) and an amino compound of formula (20) to obtain an amide compound and
a step of deprotecting at least one of P², P³ and P⁴ in the amide compound.

The step (d2) is a step including
a step of hydrolyzing the compound (4) to obtain a corresponding carboxylic acid compound,
a step of condensing the hydrolyzed carboxylic acid compound and an amino compound of formula (20) to obtain an amide compound, and
a step of deprotecting at least one of P², P³ and P⁴ in the amide compound.

The step (d3) is a step including
a step of deprotecting at least one of P², P³ and P⁴ in the compound (4),
a step of reacting the deprotected compound and the amino compound of formula (20) to obtain an amide compound,
a step of deprotecting at least one of P², P³ and P⁴ in the amide compound.

In the step (d), there are cases where any one or two of P², P³ and P⁴ (particularly P²) is deprotected at the same time as the conversion of the ester group into a N-substituted or unsubstituted carbamoyl group. In such a case, since additional deprotection of deprotected P among P², P³ and P⁴ is not required, only a non-deprotected P out of P², P³ and P⁴ needs to be deprotected. Such a step is the step of deprotecting at least one of P², P³ and P⁴ in the steps (d1), (d2) and (d3).

In a case where P², P³ and P⁴ are not deprotected at the same time as the conversion of the ester group into a N-substituted or unsubstituted carbamoyl group, in the steps (d1), (d2) and (d3), all of P², P³ and P⁴ are deprotected.

In the steps of deprotecting at least one of P², P³ and P⁴ in the steps (d1), (d2) and (d3), all of P², P³ and P⁴ may be collectively deprotected, may be separately deprotected or may be deprotected in two separate times.

In the step (d), there are cases where P², P³ and P⁴ are all deprotected at the same time as the conversion of the ester group into a N-substituted or unsubstituted carbamoyl group (for example, a case where a compound in which R¹ and R² are hydrogen atoms is produced by heating with ammonia water). At this time, the step of deprotecting at least one of P², P³ and P⁴ becomes unnecessary, and the step (d) is the following step (d4) or (d5).

The step (d4) is
a step of reacting the compound (4) and the amino compound of formula (20) to obtain an amide compound.

The step (d5) is a step including
a step of hydrolyzing the compound (4) to obtain a corresponding carboxylic acid compound, and
a step of condensing the hydrolyzed carboxylic acid compound and an amino compound of formula (20) to obtain an amide compound.

The compound (5) can be obtained as a solid (preferably as a crystal) by filtering a precipitated solid in a solvent and performing solid-liquid separation.

The solvent that is used in the solid-liquid separation is not particularly limited as long as the compound (5) is precipitated as a solid and a small amount of the remaining raw material or a by-product is dissolved in the solvent, and examples thereof include ether-based solvents, aliphatic hydrocarbon-based solvents, lower aliphatic acid ester-based solvents, alcoholic solvents, water and the like. An alcoholic solvent is preferable, methanol, ethanol, n-propanol or i-propanol is more preferable, and methanol is still more preferable.

Two or more of the solvents can be used in combination.

The temperature is not particularly limited as long as the compound (5) is precipitated and an impurity such as the remaining raw material or the by-product is dissolved at the temperature, and the compound can be obtained as a solid, preferably as a crystal, by, for example, stirring the compound in a solvent mixture of methanol and water and separating a precipitated solid into solid and liquid by filtration.

The stirring temperature is preferably 0 to 40°C and more preferably 20 to 30°C.

### Step (e) guanine protection step

A compound (6) can be synthesized by protecting a 2-amino group in the compound (5).

A step (e1) is a reaction step of protecting the 2-amino group of the compound (5), and a step (e2) is a step of purifying and/or isolating the compound (6) generated by the step (e1).

### Step (e1)

As a protecting group for the 2-amino group, as described above, a protecting group that is often used in organic synthetic chemistry (for example, nucleic acid chemistry) can be used, and the 2-amino group can be protected by, for example, a method described in Protective Groups in Organic Synthesis, Fourth edition, written by T. W. Greene, John Wiley & Sons Inc. (2006). Phenoxyacetyl group, 4-isopropylphenoxyacetyl group, acetyl group, benzoyl group, isobutyryl group and N,N-dimethylaminomethylene group are preferable.

When n for the compound (6) is 1, P¹ is formula (14).

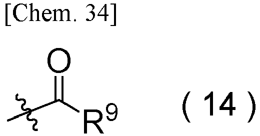

In the formula, R⁹ is a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group.

The wavy line indicates that a carbonyl group in formula (14) is bonded to a 2-amino group of a guanine base.

R⁹ is preferably methyl group, ethyl group, i-propyl group, phenyl group or phenoxymethyl group and more preferably i-propyl group or phenoxymethyl group.

The protection of the 2-amino group by an acyl-based protecting group of formula (14) (for example, isobutyryl, acetyl, benzoyl or phenoxyacetyl) can be performed by reacting a corresponding acyl halide (for example, isobutyryl chloride, acetyl chloride, benzoyl chloride or phenoxyacetyl chloride) in a solvent under a basic condition (for example, triethylamine, N',N'-diisopropylethylamine or pyridine). The solvent is not particularly limited as long as a protection reaction progresses favorably, an ether-based solvent such as tetrahydrofuran, a halogenated hydrocarbon-based solvent such as dichloromethane or a lower aliphatic acid ester-based solvent such as ethyl acetate is preferable, and ethyl acetate is more preferable.

The temperature is preferably -20 to 20°C and more preferably -10 to 10°C. The reaction time is, for example, 0.5 to 48 hours, preferably 1 to 24 hours and more preferably 1 to 6 hours.

When n for the compound (6) is 0, P¹ is formula (15).

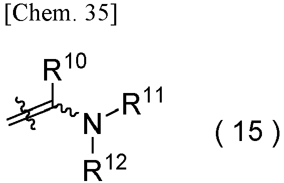

In the formula, R¹⁰ is a hydrogen atom, a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group, and R¹¹ and R¹² are the same as or different from each other and are substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, or are bonded to each other to form a ring together with the nitrogen atom to which they are bonded. The wavy line shown to intersect with the double bond indicates that the double bond is bonded to a nitrogen atom of a 2-amino group of a guanine base.

For example, when R¹⁰ is a hydrogen atom, and R¹¹ and R¹² are methyl groups, a group of formula (15) is a dimethylaminomethylene group. When R¹⁰ is a hydrogen atom, and R¹¹ and R¹² are ethyl groups, the group of formula (15) is a diethylaminoethylene group. When R¹⁰ is a hydrogen atom, and R¹¹ and R¹² are bonded to each other to form pyrrolidine together with the nitrogen atom to which they are bonded, the group of formula (15) is a 1-pyrrolidinomethylene group. Protecting groups for the amino groups in these nucleic acid bases of formula (15) are called amidinyl-based protecting groups in the nucleic acid medicine field.

R¹⁰ is preferably a hydrogen atom. R¹¹ and R¹² are the same as or different from each other and are preferably alkyl groups, more preferably methyl groups or ethyl groups, and R¹¹ and R¹² are still more preferably both methyl groups.

The wavy line in formula (15) indicates any one of a cis form and a trans form or a mixture thereof. The wavy line is preferably a trans form.

The trans form is the following formula (15-trans), and the cis form is the following formula (15-cis).

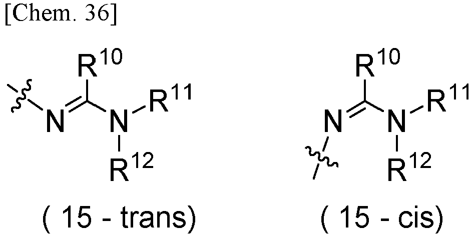

In the formula, R¹⁰, R¹¹ and R¹² are the same meaning as each reference sign in formula (15).

In formula (IS-trans), the left nitrogen atom indicates the nitrogen atom of the 2-amino group of the guanine base. In formula (15-cis), the left nitrogen atom indicates the nitrogen atom of the 2-amino group of the guanine base. The wavy lines indicate that the nitrogen atom of the 2-amino group of the guanine base is bonded to a carbon atom at a 2-position of the guanine base.

The protection of the 2-amino group by an amidinyl-based protecting group of formula (15) can be performed by reacting a corresponding alkylformamide dimethyl acetal (for example, N,N-dimethylformamide dimethyl acetal, N,N-diethylformamide dimethyl acetal, 1-(dimethoxymethyl)pyrrolidine) in a solvent. Although the solvent is not particularly limited as long as the reaction progresses favorably, an aprotic polar organic solvent such as dimethylformamide or an alcoholic solvent such as methanol is preferable, and methanol is more preferable.

The reaction temperature is preferably 20 to 50°C and more preferably 30 to 40°C.

The reaction time is, for example, 0.5 to 48 hours, preferably 1 to 24 hours and more preferably 1 to 12 hours.

If necessary, it is possible to temporarily protect the 3' and 5' hydroxy groups and then introduce a protecting group into the 2-amino group. Examples of the temporary protecting group for the 3' and 5' hydroxy groups include silyl-based protecting groups (for example, trimethylsilyl group, tert-butyldimethylsilyl group, triisopropylsilyl group and the like).

### Step (e2)

The compound (6) is heated and stirred in a solvent and then cooled, a precipitated solid is filtered and separated into solid and liquid, whereby a solvate of the solvent can be obtained as a solid, preferably as a crystal. During heating and stirring, the compound (6) may fully dissolve or may only partially dissolve.

The solvent that is used in the solid-liquid separation is not particularly limited as long as the solid (preferably crystal) of the solvate can be obtained in the solvent, and examples thereof include ether-based solvents, aliphatic hydrocarbon-based solvents, lower aliphatic acid ester-based solvents, alcoholic solvents, water and the like. An alcoholic solvent is preferable, methanol, ethanol, n-propanol or i-propanol is more preferable, and methanol is still more preferable.

Two or more of the solvents can be used in combination.

The solvate of the compound (6) preferably contains 0.3 to 10 mol of the solvent, more preferably contains 0.5 to 5 mol of the solvent, still more preferably contains 0.5 to 2 mol of the solvent and far still more preferably contains 0.5 to 1.2 mol of the solvent with respect to 1 mol of the compound (6).

The heating temperature is not particularly limited as long as the temperature is the solvent reflux temperature of the solvent and is, for example, 30 to 80°C. The cooling temperature is not particularly limited as long as the compound (6) or a solvate thereof is precipitated and an impurity such as the raw material remaining from the steps (a) through (e1) or a by-product is dissolved at the temperature, and examples thereof include - 40 to 20°C.

When the compound (6) is separated into the solid (preferably the crystal) of the solvate through a solid-liquid separation, it is possible to remove an impurity and to obtain a high-purity compound (6). Examples of the impurity include the raw material remaining in the steps (a) to (e), a raw material-derived by-product of each step, a reagent-derived substance and the like. For example, even in a case where the compound (1) remains in (step a), when the compound (6) is separated into the solid of the solvate through a solid-liquid separation in the step (e), it is possible to remove the compound (1) remaining in the step (a), a by-product derived from the remaining compound (1) or the like. As a result, in a method for producing the compound (6) through the steps (a) to (e) using the compound (1) as a raw material, column chromatography is reduced or column chromatography is not required, and it becomes possible to acquire a high-purity compound (6). The number of times of column chromatography purification including all of the steps (a) to (e) is, for example, 0 to 3, preferably 0 to 2, more preferably 0 or 1 and particularly preferably 0 (that is, all of the steps (a) to (e) do not include column chromatography purification). The steps (a) to (e) are continuously performed in a preferred manner. In the present specification, the steps being "continuously performed" is understood not to include column chromatography purification.

For example, in a case where the compound (6) is formula (16), 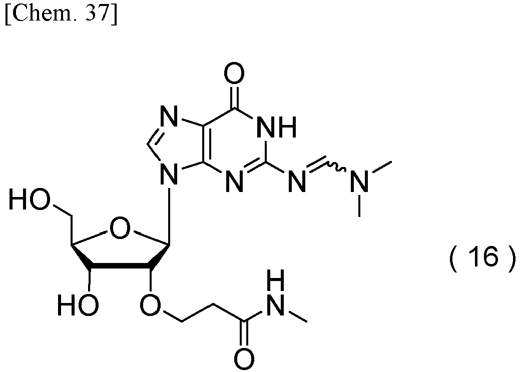 a methanol solvate can be obtained as a crystal by heating and stirring the compound in a methanol solvent, then, cooling the compound and separating a precipitated solid into solid and liquid by filtration. The temperature during heating is preferably 30 to 60°C and more preferably 35 to 45°C. The temperature during cooling is preferably -10 to 10°C and more preferably -5 to 5°C. The methanol solvate of the compound contains 0.5 to 2.5 mol of methanol, preferably contains 0.5 to 1.5 mol of methanol, more preferably contains 0.5 to 1.0 mol of methanol and particularly preferably contains 0.6 to 0.8 mol of methanol with respect to 1 mol of the compound. The geometric isomerism of the wavy line of the compound of formula (16) is preferably a trans form.

### Step (f) conversion step of 5'-position and/or 3'-position

A 3' hydroxy form of a compound (7) (that is, the compound (7) in which R⁴ is a hydrogen atom) can be synthesized by protecting a 5' hydroxy group of the compound (6).

A 3' protective form of the compound (7) (that is, the compound (7) in which R⁴ is a protecting group for a hydroxy group) can be synthesized by protecting a 3' hydroxy group of a 3' hydroxy form of the compound (7).

A 3' phosphoramidite form of the compound (7) (for example, the compound (7) in which R⁴ is formula (17), which will be described below) can be synthesized by phosphoramidizing the 3' hydroxy group of the 3' hydroxy form of the compound (7).

For the protection of the 5' hydroxy group, as described above, a protecting group that is often used in organic synthetic chemistry (for example, nucleic acid chemistry) can be used, and the 5' hydroxy group can be protected by, for example, a method described in Protective Groups in Organic Synthesis, Fourth edition, written by T. W. Greene, John Wiley & Sons Inc. (2006).

For example, when the protecting group is a triarylmethyl-based protecting group (for example, triphenylmethyl group or 4,4'-dimethoxytriphenylmethyl group), the triarylmethyl-based protecting group can be introduced into the 5' hydroxy group by reacting a corresponding triarylmethylating agent (for example, triphenylmethyl chloride or 4,4'-dimethoxytriphenylmethyl chloride) in a solvent under a basic condition (for example, triethylamine, N',N'-diisopropylethylamine or pyridine).

The solvent is required not to affect the reaction. A halogenated hydrocarbon-based solvent such as dichloromethane is preferable. The base itself can also be used as the solvent.

The reaction temperature is preferably 0°C to a solvent reflux temperature and more preferably 20 to 30°C.

The reaction time is, for example, 0.5 to 48 hours, preferably 1 to 24 hours and more preferably 1 to 4 hours.

Examples of a protecting group for the 3' hydroxy group include protecting groups that are generally used as protecting groups for nucleoside 3' hydroxy group. In addition, the protecting group may be a phosphoramidite-type group of formula (17), and 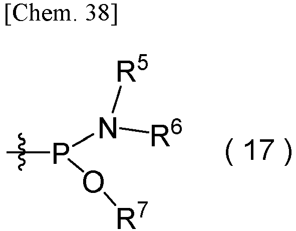 wherein R⁵ and R⁶ are the same or different C₁₋₆ alkyl groups, or R⁵ and R⁶ are bonded to each other to form a ring together with the nitrogen atom to which they are bonded, and R⁷ is a protecting group for a phosphate group.

Examples thereof include 2-cyanoethyl N,N-diisopropyl phosphoramidite of formula (18).

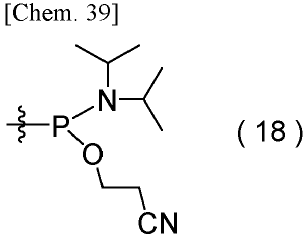

The introduction of the protecting group for the 3' hydroxy group can be performed under conditions described in, for example, Protective Groups in Organic Synthesis, Fourth edition, written by T. W. Greene, John Wiley & Sons Inc. (2006).

For example, in a case where the protecting group for the 3' hydroxy group is the phosphoramidite type (for example, 2-cyanoethyl N,N-diisopropyl phosphoramidite), the introduction of the protecting group can be performed using a phosphoramidizing agent (for example, 2-cyanoethoxy N,N,N',N'-tetraisopropyl phosphorodiamidite or 2-cyanoethoxy N,N-diisopropylchlorophosphoroamidite) under an acidic condition (for example, 4,5-dicyanoimidazole or 1H-tetrazole).

The solvent is required not to affect the reaction. A halogenated hydrocarbon-based solvent such as dichloromethane is preferable.

The reaction temperature is preferably 0 to 50°C and more preferably 20 to 30°C.

The reaction time is, for example, 0.5 to 48 hours, preferably 1 to 36 hours and more preferably 4 to 24 hours.

### Examples

Hereinafter, examples will be described, and the present invention will be described in more detail. The present invention is not limited to these examples.

In reference synthesis examples and the examples, "HPLC" means high-performance liquid chromatography, "LC-MS" means liquid chromatography-mass spectrometry, and "NMR" means nuclear magnetic resonance.

In the examples, Purif-Pack (SI-50 µm) manufactured by Shoko Science Co., Ltd. was used for purification of silica gel column chromatography.

In a case where ¹H-NMR data are described, the data are measured at 300MHz (JNM-ECP300; manufactured by JEOL Ltd. or JNM-ECX300: manufactured by JEOL Ltd.), and the chemical shifts δ (unit: ppm) of signals for which tetramethylsilane is used as the internal standard (splitting pattern, integral values) are shown. "s" means singlet, "d" means doublet, "t" means triplet, "q" means quartet, "quint" means quintet, "dd" means doublet of doublets, "m" means multiplet, "brs" means broad singlet, "brm" means broad multiplet and "DMSO-d₆" means deuterated dimethyl sulfoxide.

Unless particularly otherwise described, MS was measured using an electrospray ionization (ESI) method under the following conditions. "ESI⁺" means the ESI positive ion mode, and "ESI⁻" means the ESI negative ion mode.

### <LC-MS analysis conditions>

High-performance liquid chromatography: HPLC manufactured by SHIMADZU CORPORATION
Column: InfinityLab Poroshell 120 manufactured by Agilent Technologies, Inc.
EC-C18 (1.9 µm, 2.1 × 50 mm)
Column oven temperature: 40°C
Eluent: A: 10 mM ammonium acetate aqueous solution
B: Acetonitrile
A/B: 90/10 (0 to 1 minute), 90/10 to 5/95 (1 to 3 minutes), 5/95 (3 to 5 minutes) (volume ratio)
Eluent rate: 0.5 mL/minute
Detection wavelength: 210 nm

Unless particularly otherwise described, the HPLC relative purity was measured under the following conditions and was calculated by an area percentage method.

### <HPLC analysis conditions>

High-performance liquid chromatography: HPLC manufactured by SHIMADZU CORPORATION
Column: Hydrosphere C18 (5 µm, 4.6 × 250 mm) manufactured by YMC CO., LTD. Column oven temperature: 40°C
Eluent: A: 10 mM ammonium acetate aqueous solution
B: Acetonitrile
A/B: 95/5 (0 to 15 minute), 95/5 to 5/95 (15 to 25 minutes), 5/95 (25 to 30 minutes) (volume ratio)
Eluent rate: 1.2 mL/minute
Detection wavelength: 254 nm

Unless particularly otherwise described, powder X-ray diffraction was measured under the following conditions.

### <X-ray diffraction conditions>

X-ray diffractometer: MiniFlex600 manufactured by Rigaku Corporation
Radiation source: Cukα
Measurement range: 2θ, 3° to 40°

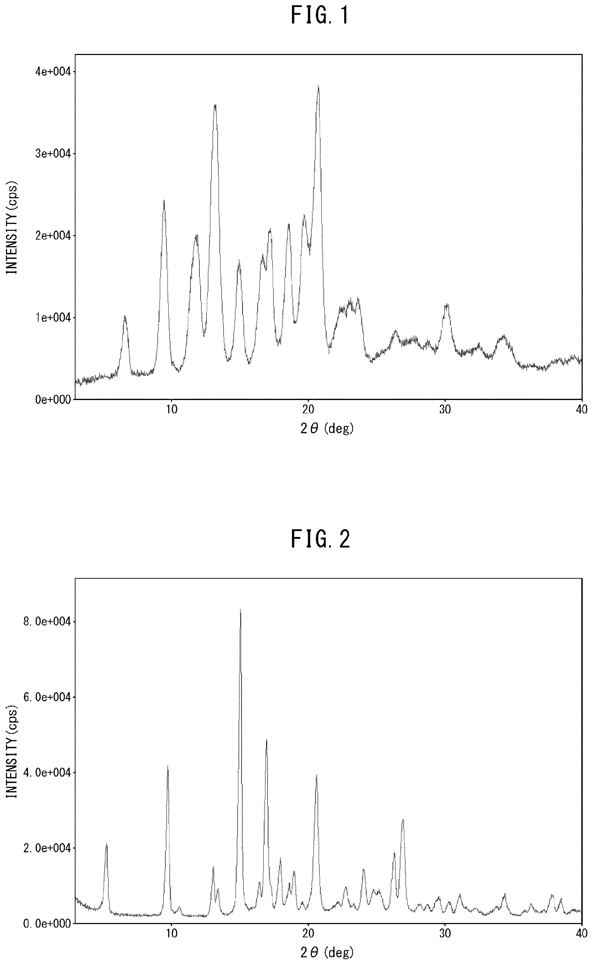

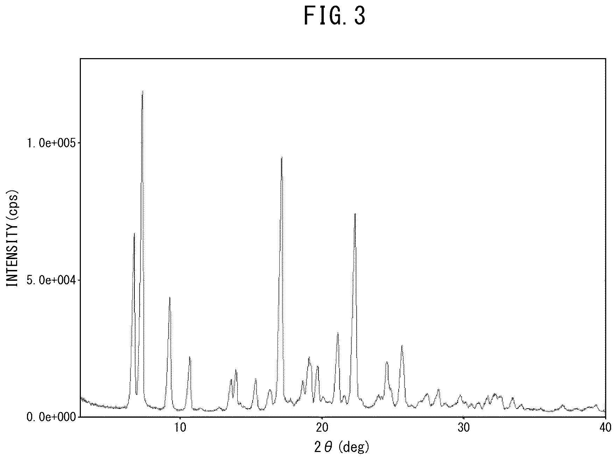

The intensity of powder X-ray diffraction is indicated by "counts per second (cps)", and it is indicated that the intensity becomes stronger as the value becomes larger. For example, "4e + 004" in the scale of the vertical axis in Fig. 1 means 4 × (10⁴), "8.0e + 004" in the scale of the vertical axis in Fig. 2 means 8.0 × (10⁴), and "1.0e + 005" in the scale of the vertical axis in Fig. 3 means 1.0 × (10⁵).

### [Example 1]

N²-Isobutyryl-2'-O-(2-methoxycarbonylethyl)-3',5'-O-(1,1,3,3-tetraisopropyldisiloxane-1,3-diyl)-2-aminoadenosine

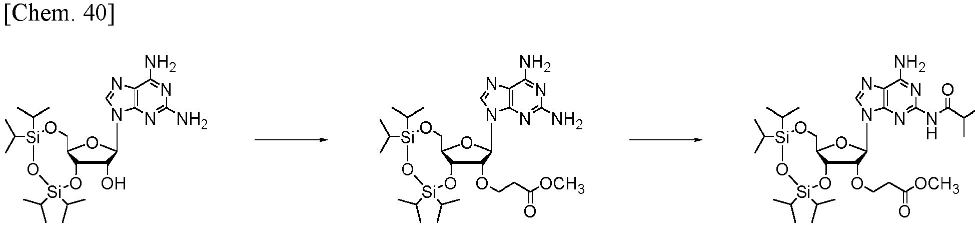

Step 1) Cesium carbonate (20.5 g, 62.9 mmol), tert-butyl alcohol (149 g) and methyl acrylate (77 ml) were added to 3',5'-O-(1,1,3,3-tetraisopropyldisiloxane-1,3-diyl)-2-aminoadenosine (29.9 g, 59.6 mmol), which is known by publications. The mixture was stirred at room temperature for 19 hours, ethyl acetate was added thereto, and the mixture was washed with a 20 wt% ammonium chloride aqueous solution and 5 wt% brine in this order. The obtained organic layer was dried over magnesium sulfate and then concentrated to obtain 2'-O-(2-methoxycarbonylethyl)-3',5'-O-(1,1,3,3-tetraisopropyldisiloxane-1,3-diyl)-2-aminoadenosine (49.8 g) as oil.

Step 2) Ethyl acetate (160 g) and pyridine (15.9 g, 200 mmol) were added to 2'-O-(2-methoxycarbonylethyl)-3',5'-O-(1,1,3,3-tetraisopropyldisiloxane-1,3-diyl)-2-aminoadenosine (49.8 g) and cooled with ice. After that, isobutyryl chloride (11 ml, 104 mmol) was added dropwise thereto under cooling with ice. The mixture was stirred for 1.5 hours under cooling with ice, and then washed with a 5 wt% sodium hydrogen carbonate aqueous solution and 5 wt% brine in this order. The obtained organic layer was dried over magnesium sulfate and then concentrated to obtain a white solid (39.3 g).

To the white solid (5.00 g) were added methanol (25 ml) and 1,8-diazabicyclo[5.4.0]-7-undecene (0.20 g, 1.3 mmol) at room temperature in order. The mixture was stirred for two hours at room temperature, and acetic acid (0.08 g, 1.3 mmol) and water (12 ml) were added thereto at room temperature in order. The mixture was stirred for one hour at room temperature, and then the precipitated solid was filtered and washed with a mixture of methanol and water (volume ratio of methanol/water: 2/1). The obtained solid was dried under reduced pressure to obtain the title compound (3.68 g, yield: 71%) as a white solid. It was confirmed by powder X-ray diffraction that the obtained solid was a crystal. The result is shown in FIG. 1.
MASS (ESI⁺) m/z; 681
Characteristic peaks of powder X-ray diffraction; 6.7, 9.5, 11.8, 13.2, 15.0, 16.6, 17.3, 18.6, 19.7 and 20.7

### [Example 2]

### 2'-O-(2-(N-methylcarbamoyl)ethyl)guanosine

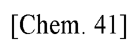

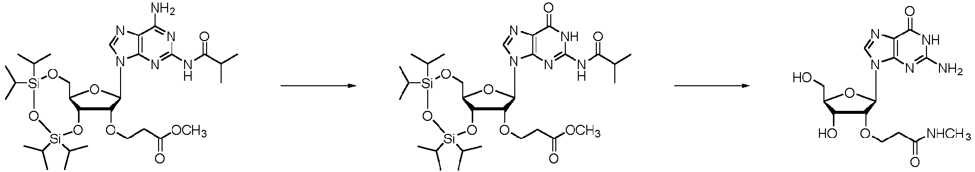

Step 1) N²-Isobutyryl-2'-O-(2-methoxycarbonylethyl)-3',5'-O-(1,1,3,3-tetraisopropyldisiloxane-1,3-diyl)-2-aminoadenosine (3.50 g, 5.14 mmol) was dissolved in acetic acid (17.5 g). The mixture was heated to 45°C, and then an aqueous solution prepared from sodium nitrite (2.18 g, 31.6 mmol) and water (3.5 g) was added dropwise thereto. The mixture was stirred at 45°C for three hours, ethyl acetate (35 g) was added thereinto, and the resultant was washed with a 50 wt% potassium hydroxide aqueous solution, a 20 wt% potassium bicarbonate aqueous solution and 10 wt% brine in this order. The organic layer was concentrated to obtain a solid of N²-isobutyryl-2'-O-(2-methoxycarbonylethyl)-3',5'-O-(1,1,3,3-tetraisopropyldisiloxane-1,3-diyl)guanosine (3.44 g).

Step 2) Methanol (6 g) and a 40 wt% methylamine-methanol solution (7 g) were added to N²-isobutyryl-2'-O-(2-methoxycarbonylethyl)-3',5'-O-(1,1,3,3-tetraisopropyldisiloxane-1,3-diyl)guanosine (3.44 g) at room temperature in order. The mixture was stirred for eight hours at room temperature, and then concentrated. To the concentrated residue was added methanol (60 g), and the resultant was concentrated to 15 g. To the solution were added triethylamine (0.67 ml, 4.8 mmol) and triethylamine trihydrofluoride (1.0 g, 5.7 mmol) at room temperature in order. The mixture was stirred overnight at room temperature, and then methoxytrimethylsilane (1.8 ml, 13 mmol) was added thereto at room temperature. The mixture was stirred for one hour at room temperature, and then concentrated. To the concentrated residue was added methanol (60 g), and the resultant was concentrated to a total amount of 12 g. The precipitated solid was filtered and washed with methanol. The filtrate was dried under reduced pressure to obtain a white solid of the title compound (1.04 g, yield: 64%). It was confirmed by powder X-ray diffraction that the obtained solid was a crystal. The result is shown in FIG. 2.
MASS (ESI⁺) m/z; 369
HPLC relative purity; 93%
¹H NMR (DMSO-d₆, 300 MHz) δ2.32-2.36 (2H, m), 2.55 (3H, d, J = 4.4 Hz), 3.49-3.65 (3H, m), 3.73-3.81 (1H, m), 3.89-3.93 (1H, m), 4.31-4.38 (2H, m), 5.07 (1H, t, J = 5.4Hz), 5.22 (1H, d, J = 3.4 Hz), 5.75 (1H, d, J = 6.5 Hz), 6.43-6.48 (1H, brs), 7.83- 7.90 (1H, m), 7.95 (1H, s), 10.62 (1H, s)
Characteristic peaks of powder X-ray diffraction; 5.3, 9.8, 15.1, 17.0, 20.6, 26.3 and 26.9

### [Example 3]

### N²-(N,N-dimethylaminomethylene)-2'-O-(2-(N-methylcarbamoyl)ethyl)guanosine methanol solvate

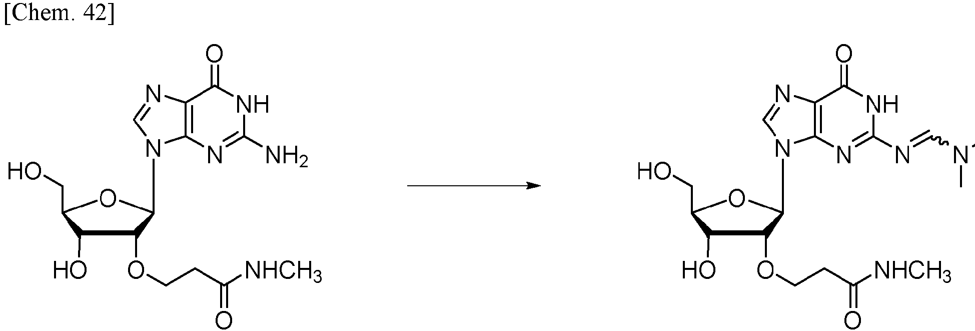

Methanol (4.2 g) was added to 2'-O-(2-(N-methylcarbamoyl)ethyl)guanosine (0.81 g, 2.3 mmol) and heated up to near 35°C. To the suspension was added N,N-dimethylformamide dimethyl acetal (0.83 g, 7.0 mmol) at 35°C. The mixture was stirred at 35°C for one hour, and then concentrated under reduced pressure. To the concentrated residue was added methanol (35 g), and the resultant was concentrated under reduced pressure. To the concentrated residue was added methanol to adjust the total amount to 8.5 g, and the resultant was cooled from 35°C to 0°C. The precipitated solid was filtered and washed with methanol. The obtained solid was dried under reduced pressure to obtain a white solid of a methanol solvate (0.86 g, yield: 84%) of the title compound. As a result of NMR measurement, approximately 0.64 equivalents of methanol was contained. In addition, the geometric isomerism of a dimethylaminomethylene group in a guanine base was a trans form. It was confirmed by powder X-ray diffraction that the obtained solid was a crystal. The result is shown in FIG. 3.
MASS (ESI⁺) m/z; 424
HPLC relative purity; 95%
¹H NMR (DMSO-d₆, 300 MHz) δ2.34 (2H, t, 6.3 Hz), 2.54 (3H, d, 4.8 Hz), 3.03 (3H, s), 3.16 (3H, s), 3.17 (1.9H, d, 4.8 Hz, methanol), 3.50-3.81 (4H, m), 3.91-3.94 (1H, m), 4.10 (0.64H, t, 5.2 Hz, methanol), 4.32-4.36 (1H, m), 4.38-4.41 (1H, m), 5.08 (1H, t, 5.6 Hz), 5.27 (1H, d, 3.9 Hz), 5.85 (1H, d, 6.0 Hz), 7.84-7.89 (1H, brm), 8.07 (1H, s), 8.56 (1H, s), 11.32-11.37 (1H, brs)
Characteristic peaks of powder X-ray diffraction; 6.8, 7.4, 9.4, 10.8, 17.2, 21.2, 22.4 and 25.7

### [Example 4]

### N²-(N,N-dimethylaminomethylene)-5'-O-(4,4'-dimethoxytrityl)-2'-O-(2-(N-methylcarbamoyl)ethyl)guanosine 3'-O-(2-cyanoethyl N,N-diisopropylphosphoamidite)

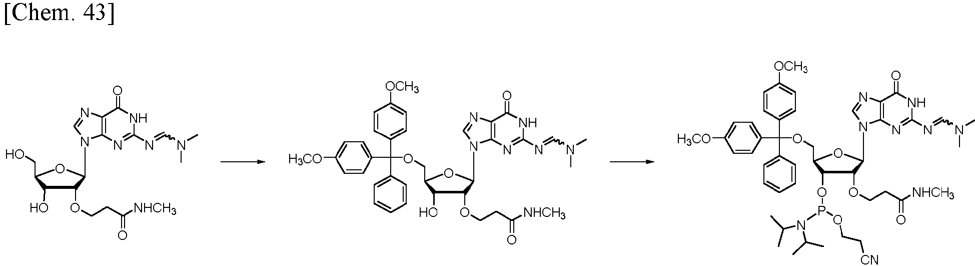

Step 1) Pyridine (100 g) was added to N²-(N,N-dimethylaminomethylene)-2'-O-(2-(N-methylcarbamoyl)ethyl)guanosine·methanol solvate (10.0 g, 22.5 mmol, containing approximately 0.64 equivalents of methanol) and concentrated under reduced pressure. To the concentrated residue was added pyridine (29 g). To the solution was added a pyridine (52 g) solution of 4,4'-dimethoxytrityl chloride (42 g, 124 mmol) dropwise at room temperature. The mixture was stirred for three hours at room temperature, and then concentrated under reduced pressure. To the concentrated residue was added chloroform (100 g), and the resultant was washed with a 5 wt% sodium hydrogen carbonate aqueous solution twice and with water once. After concentrating the organic layer, the concentrate was loaded onto a silica gel chromatography and eluted with chloroform/isopropanol (9/1 to 1/1, volume ratio) containing 0.1 vol% of triethylamine to obtain N²-(N,N-dimethylaminomethylene)-5'-O-(4,4'-dimethoxytrityl)-2'-O-(2-(N-methylcarbamoyl)ethyl)guanosine (13.8 g, yield: 84%). It was confirmed from NMR measurement that the obtained compound was a single isomer.

Step 2) N²-(N,N-dimethylaminomethylene)-5'-O-(4,4'-dimethoxytrityl)-2'-O-(2-(N-methylcarbamoyl)ethyl)guanosine (25.0 g, 34.5 mmol) was dissolved in dichloromethane (250 ml) and acetonitrile (50 ml) and cooled with ice. To the solution were added 2-cyanoethoxy N,N,N',N'-tetraisopropyl phosphorodiamidite (15.6 g, 51.8 mmol) and 4,5-dicyanoimidazole (3.1 g, 26 mmol) sequentially under cooling with ice. The mixture was stirred overnight at room temperature, and then concentrated under reduced pressure. To the concentrated residue was added chloroform (250 ml), and the resultant was washed with a 5 wt% sodium hydrogen carbonate aqueous solution twice and with water once. After concentrating the organic layer, the concentrate was loaded onto a silica gel chromatography and eluted with chloroform/acetone (9/1 to 1/9, volume ratio) containing 1 vol% of triethylamine to obtain a slightly yellow solid of the title compound (25.7 g, 80%). Since it was confirmed from NMR measurement that the geometric isomerism of the dimethylaminomethylene group in the guanine base was single, the geometric isomerism of the part was determined as a trans form.
MASS (ESI⁺) m/z; 926

### INDUSTRIAL APPLICABILITY

The present invention provides a novel method for producing a nucleoside or a nucleotide.

## Claims

1. A method for producing a protected guanosine compound of the following formula (6): 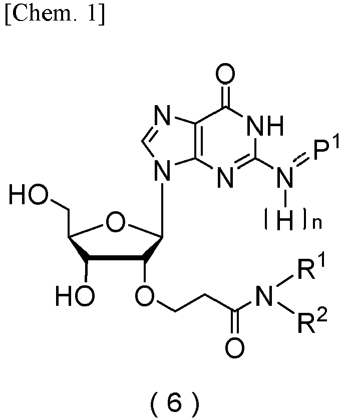
wherein n is 0 or 1,
when n is 1, P¹ is a protecting group bonded to a nitrogen atom through a single bond, when n is 0, P¹ is a protecting group bonded to a nitrogen atom through a double bond,
R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups,
the method comprising steps (e1) and (e2):
(e1) a step of converting a guanosine compound of the following formula (5) into a protected guanosine compound of formula (6) in a solvent, 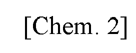 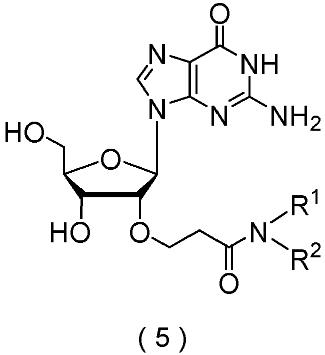 wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups,
(e2) a step of isolating a solvate of the protected guanosine compound as a solid by solid-liquid separation.

2. The production method according to claim 1, further comprising a step of isolating the solvate of the protected guanosine compound of the formula (6) as a crystal by solid-liquid separation.

3. The production method according to claim 2, wherein the solvate is a solvate of an organic solvent.

4. The production method according to claim 3, wherein the organic solvent is methanol, ethanol, n-propanol or i-propanol.

5. The production method according to claim 4, wherein the organic solvent is methanol.

6. The production method according to claim 1, wherein n is 1, and P¹ is formula (14): 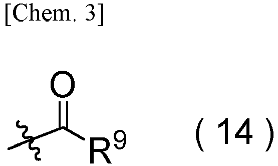 wherein R⁹ is a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group.

7. The production method according to claim 1, wherein n is 0, and P¹ is formula (15): 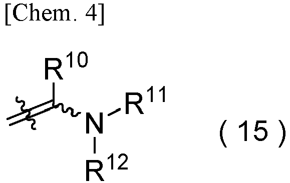 wherein
R¹⁰ is a hydrogen atom, a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group, and
R¹¹ and R¹² are the same as or different from each other and are substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, or are bonded to each other to form a ring together with the nitrogen atom to which they are bonded.

8. The production method according to claim 7, wherein R¹⁰ is a hydrogen atom, and R¹¹ and R¹² are the same as or different from each other and are C₁₋₆ alkyl groups.

9. The production method according to claim 7, wherein R¹⁰ is a hydrogen atom, and R¹¹ and R¹² are methyl groups.

10. The production method according to claim 1, wherein,
when n is 1, P¹ is an acetyl group, a phenoxyacetyl group, a benzoyl group or an i-butyryl group, and
when n is 0, P¹ is a N,N-dimethylaminomethylene group.

11. The production method according to claim 1, wherein at least one of R¹ and R² is a hydrogen atom.

12. The production method according to claim 1, wherein R¹ is a hydrogen atom, and R² is a methyl group.

13. The production method according to claim 1, wherein R¹ and R² are methyl groups.

14. The production method according to claim 12, wherein R¹ is a hydrogen atom, R² is a methyl group, n is 0, and P¹ is a N,N-dimethylaminomethylene group.

15. The production method according to any one of claims 1 to 14, further comprising the following steps (c) and (d):
(c) a step of oxidizing a 2-aminoadenosine compound of formula (3): 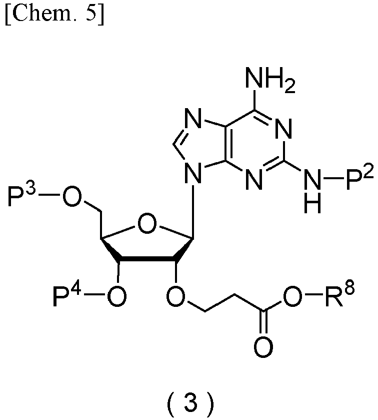
wherein P² is a protecting group for an amino group, P³ and P⁴ are the same as or different from each other and are protecting groups for a hydroxy group, and R⁸ is a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group,
to convert into a guanosine compound of formula (4): 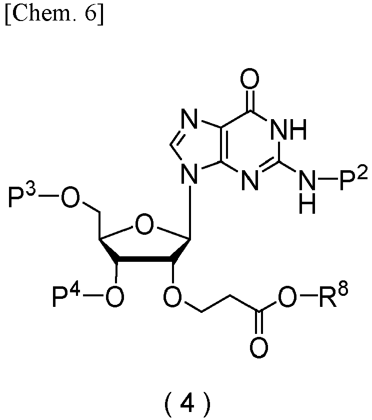
wherein P², P³, P⁴ and R⁸ are the same definition as the one defined in formula (3),
(d) a step of converting the guanosine compound of formula (4) into a compound of formula (5): 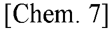 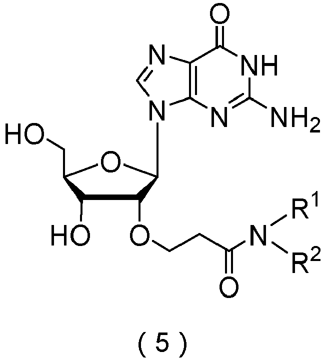 wherein R¹ and R² are the same definition as the one defined in formula (5).

16. The production method according to claim 15, wherein the step (d) is any of a step (d1), (d2) or (d3):
(d1) a step including:
a step of reacting the compound of formula (4) and an amino compound of the following formula (20) to obtain an amide compound, 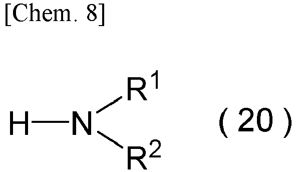
wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, and
a step of deprotecting at least one of P², P³ and P⁴ in the amide compound,
(d2) a step including:
a step of hydrolyzing the guanosine compound of formula (4) to obtain a carboxylic acid compound,
a step of condensing the hydrolyzed carboxylic acid compound and an amino compound of formula (20) to obtain an amide compound, 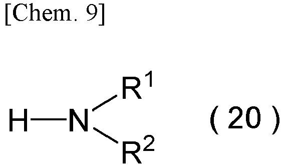
wherein R¹ and R² are the same definition as the one defined in formula (20).
a step of deprotecting at least one of P², P³ and P⁴ in the amide compound,
(d3) a step including:
a step of deprotecting at least one of P², P³ and P⁴ of the compound of formula (4),
a step of reacting the deprotected compound and the amino compound of formula (20) to obtain an amide compound, 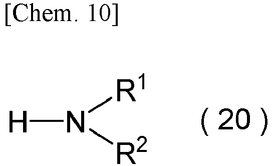
wherein R¹ and R² are the same definition as the one defined in formula (20).
a step of deprotecting at least one of P², P³ and P⁴ in the amide compound.

17. The production method according to claim 15, wherein P³ and P⁴ together form the following formula (19): 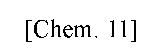 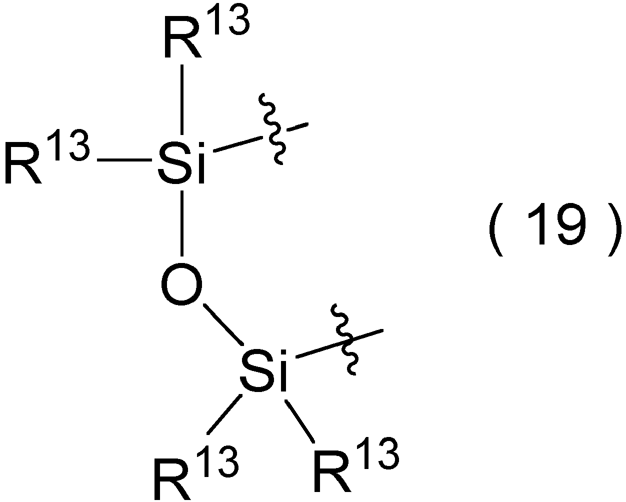 wherein R¹³s are the same as or different from one another and are substitutable C₁₋₆ alkyl groups or substitutable phenyl groups.

18. The production method according to claim 17, wherein R¹³ is an i-propyl group.

19. The production method according to claim 15, wherein R⁸ is a methyl group, an ethyl group, a n-propyl group or an i-propyl group.

20. The production method according to claim 15, wherein P² is an acetyl group, a propionyl group, an i-butyryl group, a pivaloyl group, a n-butyryl group, a benzoyl group or a phenoxyacetyl group.

21. The production method according to claim 15, wherein P² is an i-butyryl group.

22. The production method according to claim 15, further comprising the following step (b):
(b) a step of protecting a 2-amino group of a 2-aminoadenosine compound of the following formula (2) to convert into a compound of formula (3), 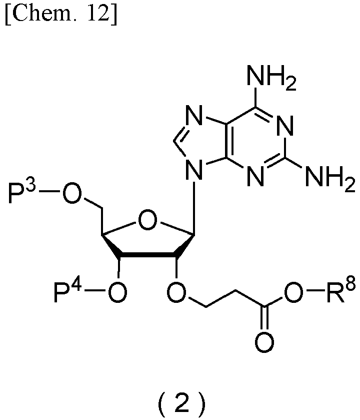
wherein P³ and P⁴ are the same as or different from each other and are protecting groups for a hydroxy group, and R⁸ is a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group.

23. The production method according to claim 22, further comprising the following step (a):
(a) a step of reacting an acrylic acid ester of the following formula (9) with the 2-aminoadenosine compound of formula (1) to convert into the 2-aminoadenosine compound of formula (2), 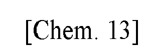 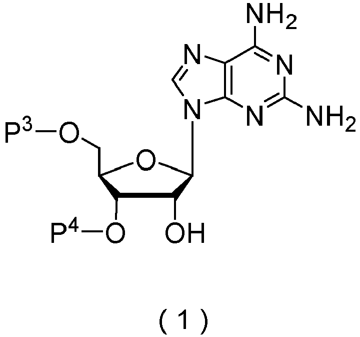
wherein P³ and P⁴ are the same as or different from each other and are protecting groups for a hydroxy group, 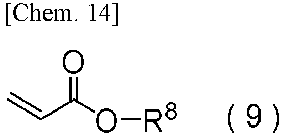
wherein R⁸ is a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group, 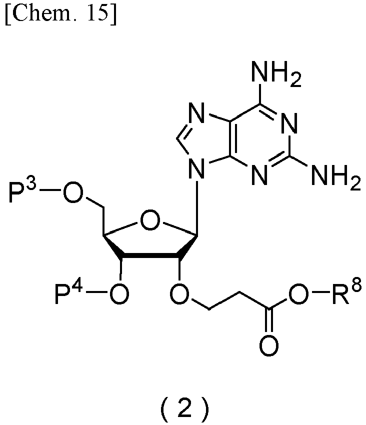
wherein P³ and P⁴ are the same as or different from each other and are protecting groups for a hydroxy group, and R⁸ is a substitutable C₁₋₆ alkyl group, a substitutable C₇₋₁₆ aralkyl group, a substitutable C₂₋₆ alkenyl group, a substitutable C₂₋₆ alkynyl group or a substitutable C₆₋₁₄ aryl group.

24. The production method according to claim 23, wherein the step (a), the step (b), the step (c), the step (d), the step (e1) and the step (e2) as a whole include a column purification step once to three times, or do not include the column purification step.

25. The production method according to claim 24, wherein the step (a), the step (b), the step (c), the step (d), the step (e1) and the step (e2) are continuously performed.

26. A method for producing a guanosine compound of formula (7): 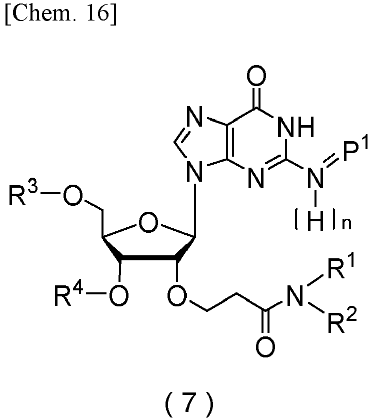
wherein n is 0 or 1,
when n is 1, P¹ is bonded to a nitrogen atom through a single bond,
when n is 0, P¹ is a protecting group bonded to a nitrogen atom through a double bond,
R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups,
R³ is a protecting group for a hydroxy group, and
R⁴ is a hydrogen atom, a protecting group for a hydroxy group or a group of formula (17): 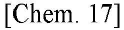 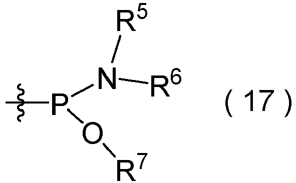
wherein R⁵ and R⁶ are the same as or different from each other and are C₁₋₆ alkyl groups, or R⁵ and R⁶ are bonded to each other to form a ring together with the nitrogen atom to which they are bonded, and R⁷ is a protecting group for a phosphate group,
the method comprising:
a step of converting a guanosine compound of the following formula (5) into a protected guanosine compound of the following formula (6), 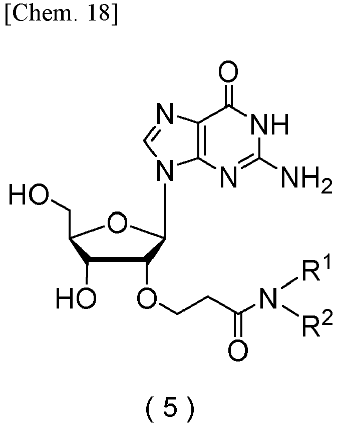
wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, 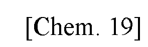 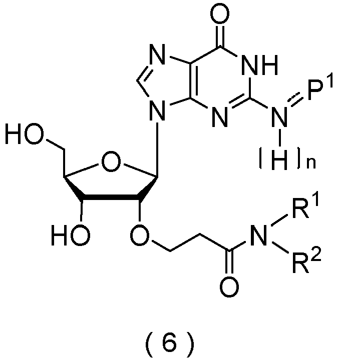
wherein n is 0 or 1,
when n is 1, P¹ is bonded to a nitrogen atom through a single bond,
when n is 0, P¹ is a protecting group bonded to a nitrogen atom through a double bond,
R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups;
a step of isolating a solvate of the protected guanosine compound as a solid; and
a step of dissolving the solvate of the protected guanosine compound in a solvent and protecting a 5'-position hydroxy group.

27. The production method according to claim 26, wherein R³ is a 4,4'-dimethoxytriphenylmethyl group.

28. The production method according to claim 26, wherein R⁷ is a 2-cyanoethyl group.

29. The production method according to claim 28, wherein R⁵ and R⁶ are i-propyl groups.

30. A method for producing a compound of formula (11), comprising:
a step of isobutylating a 2-amino group of a compound of the following formula (21) to convert into a compound of the following formula (11); and 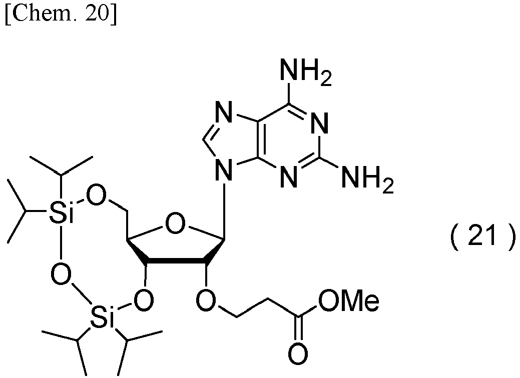 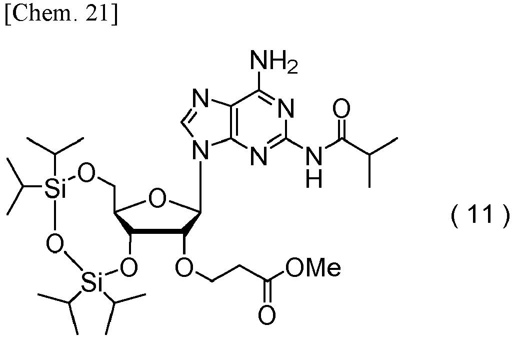
a step of isolating the compound of formula (11) as a solid by solid-liquid separation.

31. A method for producing a compound of formula (5), comprising:
a step of deprotecting an isobutyryl group and a silyl group of a compound of the following formula (11): 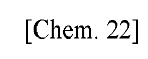 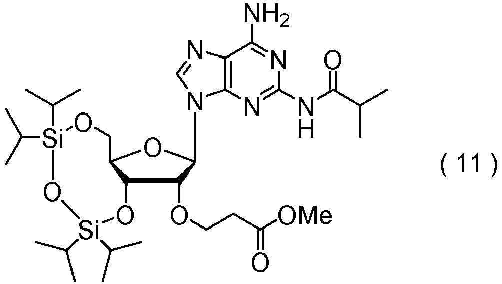
a step of adding an amino compound of the following formula (20) in a solvent to convert into a compound of the following formula (5), 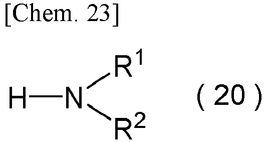
wherein R¹ and R² are the same definition as the one defined in formula (20) of claim 16, 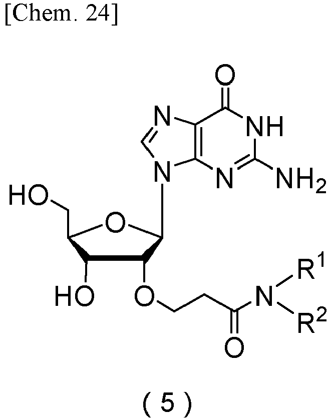
wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups; and
a step of isolating the compound of formula (5) as a solid by solid-liquid separation.
